# EUROPEAN PATENT APPLICATION

(11) **EP 1 122 312 A1**
(43) Date of publication of application: **08.08.2001**
(21) Application number: 99947862.1
(22) Date of filing: 13.10.1999
(51) Int. Cl.: C12N 15/12, C07K 14/47, C12N 5/10, G01N 33/53, C12Q 1/68, C07K 16/18, C12P 21/02

(54) **NOVEL PROTEIN WAR-1 AND GENE THEREOF**

(30) Priority: 13.10.1998 JP 29071198
(71) Applicant: Sumitomo Pharmaceuticals Company, Limited, Osaka 541-8510 (JP)
(72) Inventor: TOHDOH, Naoki, Higashinada-ku, Kobe-shi, Hyogo 658-0056 (JP); YOSHIMA, Tadahiko, Toyono-gun, Osaka 563-0105 (JP); KOMIYA, Kazuo, Nishinomiya-shi, Hyogo 662-0831 (JP); TOJO, Shinichiro, Ashiya-shi, Hyogo 659-0091 (JP); NEMOTO, Kiyomitsu, Shizuoka-shi, Sizuoka 420-0911 (JP); ISHIKAWA, Hironori, Toyonaka-shi, Osaka 561-0802 (JP); OKUYAMA, Hajime, Nishinomiya-shi, Hyogo 662-0831 (JP)
(74) Representative: VOSSIUS & PARTNER
(86) International application number: JP9905631
(87) International publication number: WO0022123

(57) **Abstract**

A novel protein having an inhibitory effect on cancer cell proliferations, WAR-1, DNA encoding WAR-1, expression vectors containing the DNA, transformants introduced with the expression vector, DNAs useful for hybridization probe or PCR primer comprising all or part of the DNA encoding WAR-1, an antibody directed to WAR-1, and uses of these substances in diagnostic and therapeutic fields are provided.

## Description

### TECHNICAL FIELD

The present invention relates to a novel protein referred to as WAR-1, and the gene thereof. More particularly, it relates to a novel protein, WAR-1, having an inhibitory effect on cancer cell proliferations, the gene encoding WAR-1, an antibody directed to WAR-1, and uses of these substances in diagnostic and therapeutic fields.

Further, the invention relates to a composition for facilitating neurotrophic factor secretions, which comprises as an active ingredient an endoplasmic reticulum membrane protein, a WAR-1 polypeptide, which is recognized to be expressed specifically in brain, or a gene encoding the same.

### BACKGROUND ART

In recent years, anti-tumor agents have been developed toward various aspects, and those agents targeting replications of cells or central dogma comprising gene transcriptions and translations, as well as those agents directed to signal transmittances, differentiation, cell cycles, metabolisms, apoptosis, telomerase, or the like, have been developed. However, decisive anti-tumor agents have not been found yet, and there is a demand for anti-tumor agents based on a new mechanism.

On the other hand, it has been known that mRNAs of proteins having a signal peptide are transported through endoplasmic reticulum (ER) membrane after the signal peptide is synthesized, and then translations occur within the ER. A factor referred to as TRAM was known as an entity responsible for the ER membrane transport (ER membrane passage) (Görlich et al., Nature, 357, 47-52, 1992).

Typically, the translation starts on binding mRNAs of proteins to be transported to the ER membrane to ribosome after being imported into the cytoplasm, and a signal sequence necessary for membrane transport is bound to a signal sequence-recognition protein. Subsequently, the complex is bound to a receptor for the signal sequence-recognition protein to be anchored on the ER membrane. The complex between the protein to be translated and the ribosome is released from the signal sequence-recognition protein, and binds to Sec61p, while TRAM resident on the ER membrane recognizes and associates with the complex. Then, the translated protein is transported into the lumen of ER through Sec61p. Such events have been understood (Jungnickel et al., Cell, 82, 261-270, 1995). It has been also known that TRAM is not always necessary for the ER membrane transport of proteins having certain signal sequences, which constitutes an exception to the mechanism for the ER membrane transport as mentioned above (Voigt et al., J. Cell Biol., 134, 25-35, 1996).

No substance that shares a homology with TRAM responsible for the ER membrane transport with respect to the amino acid sequence and the base sequence has not been reported, except for KIAA0057 gene that had been discovered in acute myeloid leukemia cells (Nomura et al., DNA Res., 1, 223-229,1994). Further, any information such as the relationship between the substances and the cancer has not been reported.

### DISCLOSURE OF THE INVENTION

The present invention aims to provide a novel protein, WAR-1, and the gene thereof. Specifically, it aims to provide a novel protein, WAR-1, whose structure shares a high homology with TRAM, and whose function involves an inhibitory effect on cancer cell proliferations; the gene encoding WAR-1; an antibody directed to WAR-1, and uses of these substances in diagnostic and therapeutic fields.

Further, the invention aims to provide a composition for facilitating neurotrophic factor secretions, which comprises as an active ingredient a WAR-1 polypeptide that an ER membrane protein, a portion thereof, or a gene encoding the same.

The inventors of the present application conducted the random screening for clones from the juvenile rat cDNA library. During the screening, the inventors successfully cloned the gene of a novel protein that shares a high homology with TRAM responsible for the ER membrane transport (the ER membrane passage) (Görlich et al., Nature, 357, 47-52, 1992) and with KIAA0057 (Nomura et al., DNA Res., 1, 223-229,1994). The inventors named the novel protein WAR-1. WAR-1, which structure shares a homology with TRAM as shown above had been unclear in terms of its function. Our continued study revealed that the expression of a WAR-1 gene in cancer cells inhibited their proliferation, and it has been found that WAR-1 of the present invention has an inhibitory effect on cancer cell proliferations. Accordingly, the pharmaceutical composition comprising as an active ingredient WAR-1 of the present invention or the gene encoding WAR-1 is believed to be useful as novel anti-cancer medicaments. In view of the fact that WAR-1 of the present invention also inhibit the proliferation of sarcomas that show high malignancies, the protein is expected to have availability in the clinical field.

As a result of the studies of the expression of the WAR-1 gene in tissues and various cancer cells, it was found that although the WAR-1 gene is not normally expressed in tissues such as liver, lung, and lymphoid tissue (spleen, thymus, and leukocyte), the malignant transformation causes specific expression of the gene. Accordingly, it is believed that a partial fragment of the WAR-1 gene or an antibody directed to WAR-1 would be used in diagnosis for these cancers.

On the other hand, in view of the fact that the gene of the protein WAR-1 responsible for the membrane transport of secretory proteins into the ER is overexpressed in the adult rat brain, the present inventors studied and discovered that enhanced expression of the gene in glial cells induces increased secretion of proteins having effects on neurite extension. Further, it was found that overexpression of the gene facilitates or accelerates the secretions of diverse neurotrophic factors produced by glial cells or nerve cells themselves.

Specifically, the present inventors demonstrated that the gene of the rat type of WAR-1 (hereinafter may be abbreviated as rWAR-1) of SEQ ID: No. 1 is expressed in not only the brain but also the retina. Further, the inventors demonstrated that the gene of the human type of WAR-1 (hereinafter may be abbreviated as hWAR-1) of SEQ ID: No. 2 is specifically expressed in not only the central nervous system but also the peripheral nervous system on the basis of the showing that the hWAR-1 gene is expressed in the whole brain and the spinal cord. Furthermore, the inventors demonstrated that neurotrophic factors having effects on neurite extension of PC12 cells derived from rat adrenal melanocytoma are expanded in culture supernatant of human glioblastoma, T98G cells, which are infected with an adenovirus vector containing the hWAR-1 gene.

The present invention has been completed on the basis of the findings as described above.

Thus, the present invention relates to:
(1) A DNA encoding a protein selected from a group consisting of:
   (a) a protein comprising the amino acid sequence of SEQ ID: No. 2 or SEQ ID: No. 4, and
   (b) a protein comprising an amino acid sequence containing deletion, substitution and/or addition of one or more amino acid residues in the amino acid sequence of SEQ ID: No. 2 or SEQ ID: No. 4, said protein having an inhibitory effect on cancer cell proliferations;
(2) A DNA selected from a group consisting of
   (c) a DNA comprising the base sequence of SEQ ID: No. 1 or SEQ ID: No. 3, and
   (d) a DNA that hybridizes with the DNA of the above (c) under stringent conditions, and encodes a protein having an inhibitory effect on cancer cell proliferations;
(3) The DNA of the above (2), which is cloned from chromosomal DNA libraries using all or part of the DNA of SEQ ID: No. 1 or SEQ ID: No. 3 as probe;
(4) The DNA of the above (3), which contains a promoter region;
(5) The DNA of the above (1) or (2), which is contained in the microorganism of deposit number FERM BP-6910 or FERM BP-6911;
(6) A protein obtainable by the expression of the DNA of any one of the above (1) to (5);
(7) A recombinant expression vector comprising the DNA of any one of the above (1) to (5);
(8) A recombinant adenovirus vector comprising the DNA of any one of the above (1) to (5);
(9) A transformant wherein the cell is transformed with the recombinant expression vector of the above (7) or (8);
(10) A DNA which is useful as hybridization probe or PCR primer, which is a single- or double-stranded DNA comprising all or pat of the DNA of any one of the above (1) to (5), and which makes possible the specific detection of the expression of a DNA comprising the base sequence of SEQ ID: No. 1 or SEQ ID: No. 3;
(11) The DNA of the above (10), which consists of the following sequences: 
5' primer sequence; 5'-CACCTGGCTGGATCGCAGAATCGG-3' (SEQ ID:
No. 7)
3' primer sequence; 5'-CTCTTTCCTCTTTGGCGGACAGTC-3' (SEQ ID:
No. 8);
(12) A method for detecting the expression of a DNA comprising the base sequence of SEQ ID: No. 1 or SEQ ID: No. 3, which comprises using the DNA of the above (10) or (11) as hybridization probe or PCR primer;
(13) An antibody that binds to the protein of the above (6);
(14) A method for detecting the expression of a protein comprising the amino acid sequence of SEQ ID: No. 2 or SEQ ID: No. 4, which comprises using the antibody of the above (13);
(15) A method for diagnosing cancers, which comprising the method for the detection of the above (12) or (14);
(16) A pharmaceutical composition comprising the DNA of any one of the above (1) to (5), or the protein of the above (6) as an active ingredient;
(17) A composition for inhibiting proliferation of cancer cells, which is characterized in that the composition enhances the expression level of a DNA comprising the base sequence of SEQ ID: No. 1 or SEQ ID: No. 3;
(18) A composition for inhibiting proliferation of cancer cells, which comprises the DNA of any one of the above (1) to (5) as an active ingredient;
(19) A composition for inhibiting proliferation of cancer cells, which comprises an adenovirus vector;
(20) A composition for facilitating neurotrophic factor secretions, which comprises as an active ingredient a DNA encoding a protein selected from a group consisting of:
   (a) a protein comprising the amino acid sequence of SEQ ID: No. 2 or SEQ ID: No. 4, and
   (b) a protein comprising an amino acid sequence containing deletion, substitution and/or addition of one or more amino acid residues in the amino acid sequence of SEQ ID: No. 2 or SEQ ID: No. 4, said protein having a facilitatory effect on neurotrophic factor secretions;
(21) A composition for facilitating neurotrophic factor secretions, which comprises as an active ingredient a DNA selected from a group consisting of:
   (c) a DNA comprising the base sequence of SEQ ID: No. 1 or SEQ ID: No. 3, and
   (d) a DNA that hybridizes with the DNA of the above (c) under stringent conditions, and encodes a protein having a facilitatory effect on neurotrophic factor secretions;
(22) The composition for facilitating neurotrophic factor secretions of the above (21), which comprises as an active ingredient a DNA that is cloned from chromosomal DNA libraries using all or part of the DNA of SEQ ID: No. 1 or SEQ ID: No. 3 as probe;
(23) The composition for facilitating neurotrophic factor secretions of the above (22), which comprises as an active ingredient the DNA that contains a promoter region;
(24) The composition for facilitating neurotrophic factor secretions of the above (20) or (21), which comprises as an active ingredient a DNA that is contained in the microorganism of deposit number FERM BP-6910, or FERM BP-6911;
(25) The composition for facilitating neurotrophic factor secretions of any one of the above (20) to (24), wherein the DNA is comprised in a recombinant expression vector;
(26) The composition for facilitating neurotrophic factor secretions of the above (25), wherein the DNA is comprised in an adenovirus vector.
(27) A composition for facilitating neurotrophic factor secretions, which comprises as an active ingredient a protein selected from a group consisting of:
   (a) a protein comprising the amino acid sequence of SEQ ID: No. 2 or SEQ ID: No. 4, and
   (b) a protein comprising an amino acid sequence containing deletion, substitution and/or addition of one or more amino acid residues in the amino acid sequence of SEQ ID: No. 2 or SEQ ID: No. 4, said protein having a facilitatory effect on neurotrophic factor secretions;
(28) A composition for facilitating neurotrophic factor secretions, which comprises as an active ingredient a protein encoded by a DNA selected from a group consisting of:
   (c) a DNA comprising the base sequence of SEQ ID: No. 1 or SEQ ID: No. 3, and
   (d) a DNA that hybridizes with the DNA of the above(c) under stringent conditions, and encodes a protein having a facilitatory effect on neurotrophic factor secretions;
(29) The composition for facilitating neurotrophic factor secretions of the above (27) or (28), which comprises as an active ingredient a protein encoded by a DNA that is contained in the microorganism of deposit number FERM BP-6910, or FERM BP-6911;
(30) A composition for facilitating neurotrophic factor secretions, which comprises as an active ingredient a substance for enhancing the expression level of the DNA of SEQ ID: No. 1 or SEQ ID: No. 3 or a substance for enhancing the production level of a protein comprising the amino acid sequence of SEQ ID: No. 2 or SEQ ID: No. 4;
(31) A pharmaceutical composition for treating neurodegenerative diseases, which comprises the composition for facilitating neurotrophic factor secretions of any one of the above (20) to (30); and
(32) A method for facilitating secretion of neurotrophic factors, which comprises enhancing the expression level of a DNA comprising the base sequence of SEQ ID: No. 1 or SEQ ID: No. 3, or enhancing the production level of a protein comprising the amino acid sequence of SEQ ID: No. 2 or SEQ ID: No. 4.

In one aspect, the present invention provides a novel protein, WAR-1, and a DNA encoding WAR-1.

In the present invention, the DNA encoding the protein is not limited to particular one as long as the DNA encodes the novel protein, WAR-1, or the DNA is similar to the DNA encoding WAR-1, and encodes a protein having an inhibitory effect on cancer cell proliferations. Specific examples include the following DNAs of (1) to (3):
1) a DNA encoding a protein comprising the amino acid sequence of SEQ ID: No. 2 or SEQ ID: No. 4, or a DNA comprising the base sequence of SEQ ID: No. 1 or SEQ ID: No. 3;
2) a DNA encoding a protein comprising an amino acid sequence containing deletion, substitution and/or addition of one or more amino acid residues in the amino acid sequence of SEQ ID: No. 2 or SEQ ID: No. 4, said protein having an inhibitory effect on cancer cell proliferations; and
3) a DNA that hybridizes with a DNA comprising the base sequence of SEQ ID: No. 1 or SEQ ID: No. 3 under stringent conditions, and encodes a protein having an inhibitory effect on cancer cell proliferations.

These DNAs are described in a sequential manner as follows.

### 1) DNA encoding WAR-1

Among the DNAs as mentioned above, "DNA encoding a protein comprising the amino acid sequence of SEQ ID: No. 2", and "DNA comprising the base sequence of SEQ ID: No. 1" are those encoding rat WAR-1 of the present invention. Further, "DNA encoding a protein comprising the amino acid sequence of SEQ ID: No. 4", and "DNA comprising the base sequence of SEQ ID: No. 3" are those encoding human WAR-1 of the present invention. Such DNAs encoding rat and human WAR-1s of the present invention have been deposited as shown below.

Specifically, *E*. *coli* DH5a (prWAR-1) that is an *E*. *coli* containing prWAR-1 wherein the DNA encoding rat WAR-1 of SEQ ID: No. 1 is incorporated into a vector pBluescript II, and E. coli DH5a (phWAR-1) that is an *E*. *coli* containing phWAR-1 wherein the DNA encoding human WAR-1 of SEQ ID: No. 3 is incorporated into a vector pBluescript II were deposited at The National Institute of Bioscience and Human Technology, Agency of Industrial Science and Technology (1-1-3 Higashi, Tsukuba, Ibaraki, Japan) under deposit numbers FERM P-17018 and FERM P-17019, respectively (deposition date: October 6, 1998 in each case). Subsequently, they were converted to international deposition (deposit numbers: BP-6910 and BP-6911; date of conversion to international deposition: October 7, 1999 in each case).

### 2) DNA encoding altered form or variant of WAR-1

Among the DNAs as mentioned above, "DNA encoding a protein comprising an amino acid sequence containing deletion, substitution and/or addition of one or more amino acid residues in the amino acid sequence of SEQ ID: No. 2 or SEQ ID: No. 4, said protein having an inhibitory effect on cancer cell proliferations" are those DNAs that encodes a protein having an inhibitory effect on cancer cell proliferations, among DNAs encoding altered forms (altered proteins) that are artificially prepared, or variants existing in the living body.

Those skilled in the art would readily prepare DNAs encoding the altered forms as described above by, for example, methods using restriction enzymes or nucleases, the site-directed mutagenesis (W.Ito et al., Gene, 102, 67-70 (1991), or the PCR method (Molecular Cloning,2nd Edt. Cold Spring Harbor Laboratory Press (1989)).

In this context, the number of amino acid residues to be subject to "deletion, substitution and/or addition" is in the range that deletion, substitution and/or addition can be achieved by the well-known methods such as site-directed mutagenesis as mentioned above.

Further, it is possible to prepare DNAs encoding the altered forms by exposing cells to mutagens without the genetic engineering approaches as mentioned above.

DNAs encoding the variants as mentioned above refer to those naturally occurring in the living body. Specifically, the deletion, substitution or addition of base or amino acid may be caused by natural events such as cancers or species specificity, and DNAs encoding such naturally occurring variants are also fallen within the DNAs of the present invention as long as those DNAs encode proteins having inhibitory effects on cancer cell proliferations.

Determination whether or not the DNAs containing alterations as shown above encode a protein having an inhibitory effect on cancer cell proliferations may be conducted by the following procedures.

Specifically, a candidate DNA for those of the present invention, such as the DNAs containing the alteration as mentioned above is incorporated into an expression vector, and the resultant vector is transformed into a cancer cell line. As a cancer cell line, human glioblastoma such as T98G is preferably used. Expression vectors may be either non-viral vectors or viral vectors, and are not limited to particular vectors as long as they can be expressed in cancer cell lines from human (the details about the expression vectors are described hereinafter). After transforming the recombinant vectors into the cancer cell lines, and culturing the same, the cell counts and morphologic change of the cells are examined. At this time, it is important to compare them with those of cells prepared as a control in accordance with the same procedure except for the use of an expression vector without the incorporated foreign DNAs. When observing that cell count is decreased, or morphology of the cell is changed compared to the control cells, then the candidate DNA may be determined to be one encoding a protein having an inhibitory effect on cancer cell proliferations.

Additionally, determination whether or not an inhibitory effect on cancer cell proliferations is exhibited may be conducted by, for example, measuring a decrease in ³H-labeled thymidine incorporation in the gene-transferred cells as mentioned above (Nagase et al., Int. J. Cancer, 65, 620-626, 1996), or measuring a decrease in tumorigenicity observed in nude mice inoculated with cancer cells that have been infected with an adenovirus having a candidate DNA as mentioned above (Cheney et al., Cancer Res., 58, 2331-2334, 1998).

### 3) DNA that hybridizes with the WAR-1 DNA under stringent conditions

Among the DNAs as described above, "DNA that hybridizes with a DNA comprising the base sequence of SEQ ID: No. 1 or SEQ ID: No. 3 under stringent conditions, and encodes a protein having an inhibitory effect on cancer cell proliferations" refers to DNAs that encode a protein having an inhibitory effect on cancer cell proliferations, among DNAs that hybridize with the rat or human WAR-1 DNA comprising the base sequence of SEQ ID: No. 1 or SEQ ID: No. 3 under stringent conditions, such as
A) a cDNA encoding WAR-1s from all vertebrates, or a cDNA encoding a partial protein of WAR-1s, and
B) a chromosomal DNA encoding WAR-1s from all vertebrates.

In this context, "DNA that hybridizes under stringent conditions" refers to those DNAs that continue to be hybridized under such a condition that the hybridization is conducted at 42°C overnight using, as hybridization buffer, a solution of a composition of 0.1% SDS, 50% formamide, 5xSSC, 1xDenhardt regent, and 250 µg/ml salmon sperm DNA; followed by washing with 2xSSC for an hour at room temperature, with 2xSSC, 0.1% SDS for 30 minutes at room temperature, and then with 0.1xSSC, 0.1%SDS at 50-65°C for 30 minutes.

The DNA of the above (A) may be cloned by, for example, hybridization using as a probe all or part of the DNA of SEQ ID: No. 1 or SEQ ID: No. 3, or by PCR using as a primer a part of DNA of SEQ ID: No. 1 or SEQ ID: No. 3. Particular procedures of the preparation of cDNA library, hybridization, PCR, selection for positive colony, sequencing of the base sequence, or the like are well known, and may be conducted according to documents such as Molecular Cloning 2nd Edt., Cold Spring Harbor Laboratory Press (1989). An example for the particular cloning procedure is provided below.

The DNA of the above (A) may be isolated by a process comprising the steps (a) and (b) for example:
a) preparing total RNA from tissues derived from a desired species or cultured cell lines, and purifying the poly(A) RNA so as to provide a cDNA library; and
b) hybridizing the cDNA library prepared in the above (a) with a probe prepared to comprise all or part of the base sequence of SEQ ID: No. 1 or SEQ ID: No. 3, thus isolating an intended DNA.

In this context, the preparation of total RNA in the above step (a) may be conducted according to usual manners that include for example treating cells with surfactants such as SDS, NP-40, Triton-X100 or in the presence of phenol, so as to effect cytolysis. Further, the preparation of total RNA may be also conducted by means of destroying cells via physical means such as using a homogenizer, treating the cell with guanidine thiocyanate, and then precipitating total RNA via cesium chloride density gradient centrifugation, or by means of treating the cell with guanidine thiocyanate, and with phenol in an acidic condition (acidic guanidine thiocyanate-phenol chloroform). Subsequently, affinity columns such as oligo(dT)-cellulose and polyU-cellulose bound with polyU are used to purify poly(A)RNA (mRNA) from the total RNA obtained by any one of the above methods. Alternatively, in cases that the length of the mRNA is known, or fractions based on the length of the mRNA are intended, sucrose density gradient centrifugation, agarose gel electrophoresis, gel filtration on columns, or the like may be used. Preparation of cDNA libraries from the mRNA obtained as described above may be conducted by synthesizing a single-stranded cDNA using the mRNA as a template, preparing a double-stranded cDNA, incorporating the same into suitable vectors, and transforming *E*. *coli* hosts with the vectors. As vectors, plasmids and λ phage vectors are often used. Details on the preparation of the cDNA libraries are provided below.

First, a single-stranded cDNA complementary to the mRNA is synthesized with reverse transcriptase (from avian myeloblastic leukemia virus; AMV, or from murine leukemia virus; Mo-MLV) using mRNA template and oligo (dT) primers attached with or without a suitable sequence at the end, or random primers consisting of six bases. Then, the mRNA is decomposed with alkaline conditions, and then a double-stranded cDNA is synthesized with reverse transcriptase or DNA polymerase using the single-stranded cDNA as template. Alternatively, double-stranded cDNA may be directly synthesized using RNase H and *E*. *coli* DNA polymerase I. In both cases, subsequently, one of enzymes such as S1 nuclease, T4 DNA polymerase, and *E*. *coli* DNA polymerase (the Klenow fragment) is used to form a blunt end at both terminuses of the synthesized double-stranded cDNA. Terminal modification is conducted on the resultant blunted double-stranded cDNA by attaching chemically-synthesized DNAs such as linker and adaptor, or dG or dC strand to the cDNA with deoxy terminal transferase so as to be ready for the incorporation of the cDNA into a suitable vector. The double-stranded cDNA is incorporated into a suitable vector, and then the vector is transformed into *E*. *coli* to prepare cDNA libraries. In case of the transformation of *E*. *coli* hosts with plasmid vector incorporated with double-stranded cDNA, the transformation may be conducted on the harvested competent cells to be transferred with the DNA at logarithmic growth phase according to the procedure discussed by Hanahan (J.Mol.Biol.,166,557 (1983)). In case of the transformation of *E*. *coli* hosts with phage vector incorporated with double-stranded cDNA, the transformation may be conducted by introducing DNAs ligated with T4 DNA ligase into phage particle via *in vitro* packaging system, and infecting *E*. *coli* hosts with the phage.

In the next step (b), an intended DNA may be isolated by - preparing probes consisting of all or part of the base sequence of SEQ ID: No. 1 or SEQ ID: No. 3, and hybridizing the cDNA library prepared as shown above with the probes. Probes in this context may be prepared by synthesizing a suitable partial fragment of the base sequence of SEQ ID: No. 1 or SEQ ID: No. 3 with DNA synthesizers or by amplifying said partial fragment via PCR, and then labeling the DNA fragment with ³²P in accordance with conventional procedures such as nick translation and random priming labeling. Conditions for hybridization are as described above.

The DNA as mentioned above (B), namely, chromosomal DNA encoding WAR-1 may be cloned for example by hybridizing chromosomal DNA libraries prepared from tissues derived from desired species or cultured cell lines with probes having all or part of the base sequence of SEQ ID: No. 1 or SEQ ID: No. 3, or having all or part of the DNA of the above (A).

In this context, preparations of chromosomal DNAs and chromosomal DNA libraries may be conducted in accordance with usual manners. Specifically, tissues derived from desired species or cultured cell lines are processed in the presence of SDS, and RNAs and proteins that are unnecessary are decomposed with RNase and proteinase K. Then, chromosomal DNA may be purified by treatment with phenol, and subsequent ethanol precipitation or dialysis. The resultant chromosomal DNA is partially cleaved with suitable restriction enzymes, or completely digested with necessary restriction enzymes in case that the length of the fragment to be cloned is known. Fragmentation of the cleaved chromosomal DNA fragments based on the DNA length to be allowed for their incorporation into cloning vectors may be conducted by sucrose density gradient centrifugation, agarose gel electrophoresis, gel filtration on columns, or the like. The cleaved fragments are incorporated into λ phage vectors or cosmid vectors, and *in vitro* packaged, thus conducting the preparation of chromosomal DNA libraries with phages or cosmids.

The chromosomal DNA encoding WAR-1 may be cloned by infecting *E*. *coli* with the phage or cosmid library as described above, and then conducting plaque or colony hybridization in accordance with conventional procedures such as using, as probe, all or part of the WAR-1 cDNA labeled with ³²P by nick translation and random priming labeling method (See Molecular Cloning 2nd Edt., Cold Spring Harbor Laboratory Press (1989).

Chromosomal DNA as described above will contain a promoter region, which is responsible for gene expression regulation, and such chromosomal DNA that contains the promoter region may be readily cloned by the procedures as mentioned above.

Recombinant expression vectors containing the DNA of the present invention may be prepared by incorporating various DNAs of the present invention into expression vectors. Transformants of the present invention may be prepared by transforming host cells with the recombinant expression vectors.

The expression vectors include both non-viral and viral vectors, and are not limited to any particular species as long as they allow the DNA of the present invention to be transferred, and allow the proteins encoded by the DNA to be expressed. Non-viral vectors include expression plasmid vectors typically used in mammal cells, and are exemplified by pBK-CMV, pCAGGS, pcDNA3.1, pZeoSV, and the like. Transformation of non-viral expression vectors incorporated with the DNA of the present invention may be achieved by calcium-phosphate coprecipitation, methods for transferring DNA molecules using liposome (the liposome method, Lipofectin method, Lipofectamine method), electroporation, microinjection, methods for transferring DNA molecules into cells along with carriers by particle guns, and the like. Host cells include HeLa, COS1, A549, 293 cells, and the like.

For the latter viral vectors, viral vectors such as adenovirus and retrovirus are typical. Specifically, DNA or RNA viruses such as avirulent retrovirus, adenovirus, adeno-associated virus, herpesvirus, vaccinia virus, poxvirus, poliovirus, Sindbis virus, Sendai virus, SV40, human immunodeficiency virus are incorporated with the gene encoding the given protein, and the recombinant viruses are infected to cells, thus being capable of transforming the gene into cells. In this context, host cells include 293, A549, and HeLa cells.

The transformants thus obtained may be continuously cultured under an appropriate condition to express and prepare proteins from the DNA of the present invention. In this context, the term "appropriate condition" refers to a condition wherein a cultivation is conducted at 37 °C under 5%CO₂ in a culture medium suitable for respective host cell.

From the transformants thus cultured in an appropriate condition, the proteins of the present invention as described above may be isolated and purified. In this connection, preparation of crude extract of the present proteins, and purification of the present proteins may be conducted according to, for example, methods as described in "Shinseikagakujikkenn-koza 1, Tannpakushitsu I-bunnri, seisei, seishitsu", Japanese Biochemical Society Ed. 1990.

Specific examples of the proteins of the present invention obtainable as shown above include rat WAR-1 comprising the amino acid sequence of SEQ ID: No. 2, and human WAR-1 comprising the amino acid sequence of SEQ ID: No. 4.

Inhibitory effect of the proteins of the present invention on cancer cell proliferations may be determined as described below. The protein of the invention is added to the culture of cancer cells such as T98G. At this time, the protein is encapsulated into liposome or bound to lipid in order to enhance the permeability across cell membrane. Alternatively, the endoplasmic reticulum-retained sequence, Lys-Asp-Glu-Leu (KDEL), is attached to the C-terminus of the protein in order to make it possible that the protein migrated to the cytoplasm be transported into the ER. Inhibitory effect of the protein of the present invention on cancer cell proliferations may be determined by examining the cell counts and morphologic change of the cancer cells after adding the protein thus treated to the culture, and culturing the same for several days. Further, Inhibitory effect of the protein of the present invention on cancer cell proliferations may be determined by examining the lowered incorporation of ³H-labeled thymidine (Nagase et al., Int. J. Cancer, 65, 620-626, 1996).

Single- or double-stranded DNA comprising all or part of the DNA encoding the protein of the present invention may be used as hybridization probe or PCR primer to detect specifically the expression of the WAR-1 DNA in tissues of the living body or cultured cell lines. Such single- or double-stranded DNAs are not limited to any particular ones as long as they allow the specific detection of the WAR-1 mRNA that is a transcription product of WAR-1 DNA.

Specific examples of the detection include the two procedures as shown below:
1) Analytical procedures wherein PCR is conducted on the substrate of total RNA or poly(A) RNA derived from test tissues or cultured cell lines, using as PCR primer two single-stranded DNAs (sense and antisense strands) that allow the specific detection of WAR-1 mRNA; and
2) Northern blotting analyses of total RNA or poly(A) RNA derived from test tissues or cultured cell lines wherein radiolabeled single-stranded or double-stranded DNAs that allow the specific detection of WAR-1 mRNA is used as probe.

The procedures for the detections (1) and (2) as described above may be conducted on the basis of text books such as Molecular Cloning 2nd Edt., Cold Spring Harbor Laboratory Press(1989). Specific examples are shown below:

The specific example of the procedure for PCR as described in the above (1) is as follows. First of all, PCR primer that allows the specific detection of WAR-1 mRNA is synthesized according to usual manners. Next, total RNA or poly(A) RNA is prepared as describe above from test tissue or cultured cell lines, and single-stranded DNA is prepared using the RNA as template with reverse transcriptase such as MMTV-RT. Subsequently, the PCR primer as previously prepared is added, and PCR reaction is routinely conducted. Conditions for PCR reaction include, for example, 35 cycles comprising 95°C for one minute, 60°C for one minute, and 72°C for two minutes, and subsequent heating at 72°C for 10 minutes. Electrophoresis of the PCR reactions on agarose gel at various concentrations enables the detection of presence or absence of the expression of WAR-1 mRNA. The detection of WAR-1 mRNA may be conducted by *in situ* PCR method (Fernandez et al., Mol. Carcinog, 20, 317-326, 1997).

The specific example of the Northern blotting analyses as described in the above (2) is as follows. First of all, single- or double-stranded DNAs that allow the specific detection of WAR-1 mRNA is radiolabeled to prepare probes. Double-stranded DNA may be prepared by, for example, labeling the PCR reactions prepared in the procedure (1) as mentioned above with ³²P in accordance with nick translation and random priming labeling. Next, total RNA or poly(A) RNA is prepared from test tissues or cultured cell lines in accordance with a similar procedure to that of the above, and formaldehyde gel electrophoresis and blotting to the nylon membrane are routinely conducted. Hybridization of the probe as previously prepared with this membrane permits the detection of presence of absence of the expression of the WAR-1 mRNA. Conditions for the hybridization wherein double-stranded DNA is used as probe include such conditions that the hybridization is conducted at 42°C for 16-45 hours using a solution of a composition of 45%(v/v) formamide, 5xSSPE, 2xDenhardt solution, 0.5%SDS, and 20 µg/ml salmon sperm DNA, followed by washing a few times with 2xSSPE and 0.5%SDS for 10 minutes at room temperature, and then a few times with 2xSSPE, 0.5% SDS at 65°C for 20 minutes.

Specific examples of single- or double-stranded DNAs as used in the detection shown above include the following.

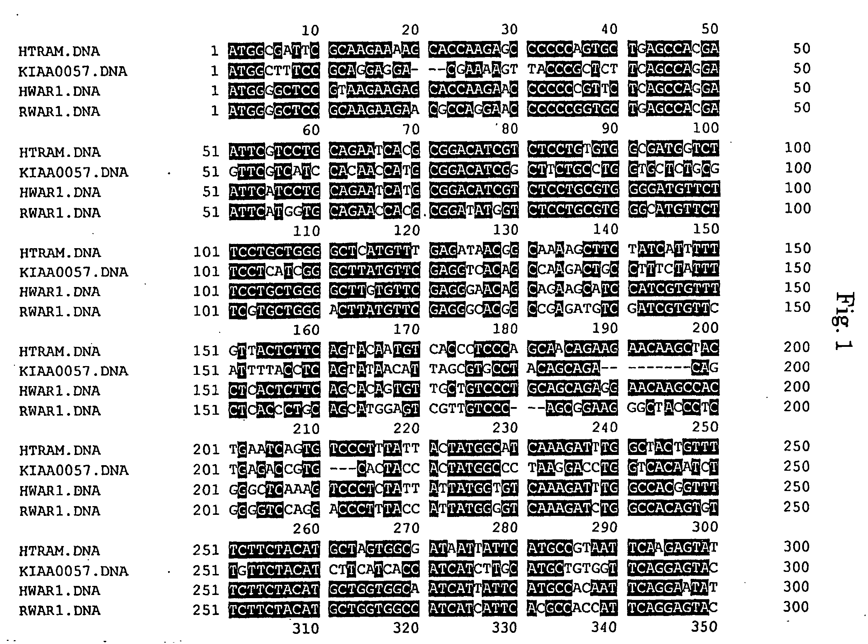

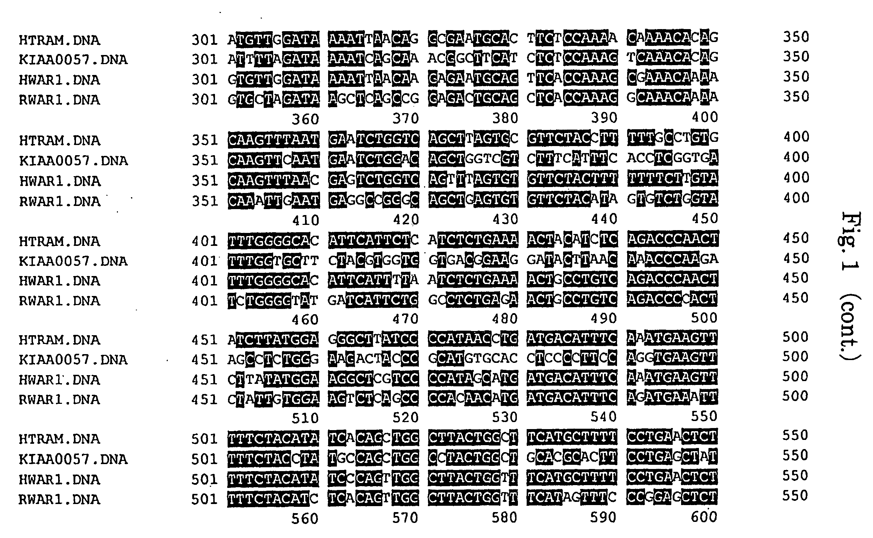

When selecting portion to be used as probe or primer specific for human WAR-1, it is important to select regions having a low homology between human TRAM and human WAR-1, consulting Figure 1 that gives comparison of the base sequences of human WAR-1 (hWAR-1) and rat WAR-1 (rWAR-1) with those of human TRAM (hTRAM) that is a known factor responsible for the ER transport and human KIAA0057 wherein the black frame shows portions sharing a homology. At this time, in order to discriminate between DNA fragments amplified from both cDNAs, the region containing deletion of partial base sequence in one of the sequences is preferably amplified. Primers specifically recognizing each of the cDNAs preferably share no homology each other in the whole primer sequences, and it is understood that the specificity would be enhanced when the sequences in the vicinity of the 3'-terminus are diversified largely each other to prohibit the elongation reaction of cDNA not intended to be amplified from proceeding. Further, design to differentiate base sequences at the 3'-terminus each other is effective for the specific amplification. Primer analysis using programs such as a software Oligo of National Biosciences may be also utilized.

On the basis of portions selected as shown above, probes of the above (1) or primers of the above (2) may be prepared in accordance with usual manners.

One example may be a single-stranded DNA of the following sequence, or a double-stranded DNA amplified by PCR reactions as shown above using the single stranded DNA as primer:

5' primer sequence; 5'-CACCTGGCTGGATCGCAGAATCGG-3' (SEQ ID:
No. 7)
3' primer sequence; 5'-CTCTTTCCTCTTGGCGGACAGTC-3' (SEQ ID:

No. 8).

SEQ ID: No. 7 corresponds to the sense strand of the sequence at positions 823-846 in hWAR-1 DNA of Figure 1, whereas SEQ ID: No. 8 corresponds to antisense strand of the sequence at positions 1093-1116 in hWAR-1 DNA of Figure 1.

The single-stranded DNA or double-stranded DNA as shown above may be used as PCR primer used in the above detection (1) or hybridization probe used in the above detection (2) to detect the specific expression of WAR-1 DNA. See Example 4 for the details. Such primer and probe may include sequences derived from native WAR-1 as well as DNA sequences containing modifications such as substitution, deletion, and addition as long as the latter allows the specific detection of WAR-1 mRNA that is a transcription product of WAR-1 DNA.

The method for detecting the WAR-1 DNA expression may be specifically used in applications to diagnosis for diseases and in examinations such as *in situ* hybridization.

As mentioned in the above description for "means for dissolving the problems", it was found that although the WAR-1 gene is not normally expressed in tissues such as liver, lung, and lymphoid tissue (spleen, thymus, and leukocyte), the malignant transformation of these tissues causes specific expression of the gene. Accordingly, the detection of the WAR-1 mRNA in cancer tissues or cancer cells derived from patients using the PCR primer or the hybridization probe specific for WAR-1 as shown above leads to diagnosis of cancers.

In the present invention, "antibody" means an antibody binding to the proteins of the present invention as shown above. Such antibodies are easily prepared, for example, according to methods described in "Antibodies: A Laboratory Manual", Lane, H. D. *et al*. eds., Cold Spring Harbor Laboratory Press (1989) and Shin-Saibokogakujikken-purotokoru, Shujun-sha (1993). Specifically, antibodies that bind to the proteins of the present invention may be prepared using the protein or parts thereof according to the present invention to appropriately immunize an animal in usual manners.

In this context, the proteins of the present invention used as immunological antigens may be obtained by introducing the recombinant expression vectors containing the DNA of the invention into *E*. *coli* or cultured cell lines, preparing a large amount of the polypeptides from the transformants, and purifying the same. Additionally, peptides comprising a partial amino acid sequence of the protein of the invention may be synthesized, and conjugated to BSA, KLH, or the like, to give immunological antigens.

Species to be immunized include any species such as rabbit, mouse, rat, chicken, bovine, donkey, ovine and horse, and may be a polyclonal or monoclonal antibody as long as those antibodies recognize the proteins of the present invention.

Such antibodies are used in detection for the expression of the WAR-1 protein, or isolation of the protein. Specifically, they may be used in an affinity chromatography, a screening for cDNA library, an immunological diagnosis, and the like.

Immunological diagnosis for cancers may be conducted using the antibodies of the present invention. Specifically, the antibodies of the invention enable to detect the cancer tissues or cells producing WAR-1, and can be applied to the diagnosis for cancers. Specific detection methods include a fluorescent antibody method, Western blotting, immunoprecipitation, and immunohistological staining. A fluorescent antibody method among them may be conducted in accordance with specific procedures described in Samoszuk et al., Am. J. Clin. Pathol., 109, 205-210, 1998 or Bernardini et al., Tumori., 83, 673-678, 1997.

A DNA or a protein of the present invention may be comprised as an active ingredient in pharmaceutical compositions. The protein of the invention has an inhibitory effect on cancer cell proliferations, as mentioned above. Accordingly, either administration of the pharmaceutical composition comprising the DNA of the invention as an active ingredient to cancer patients, said composition being used as gene therapy agents to effect gene expressions in living bodies, or administration of the pharmaceutical composition comprising the protein of the invention as an active ingredient to cancer patients enables cancer cell proliferation to be inhibited, and makes cancer therapy possible. Not only administration of the DNA or the protein of the present invention, but also administration of a factor or a compound that induces the expression of the WAR-1 gene or protein occurring in living bodies provides the above inhibitory effects on cancer cell proliferations. Accordingly, pharmaceutical compositions for inhibiting proliferation of cancer cells that contains, as an active ingredient, factors or compounds inducing the expression of the WAR-1 gene, or factors or compounds inducing the expression of the WAR-1 protein are also fallen within the scope of the present invention.

The pharmaceutical composition comprising as an active ingredient a protein of the present invention may be administered along with an adjuvant, or in a particulate dosage form. Specific examples of dosage form include liposomal preparations, particulate preparations in which the ingredient is bound to beads having a diameter of several µm, or preparations in which the ingredient is attached to lipids. The administration may be achieved in manners of sustained-release minipellet formulations. In order to transport a protein expected to occur in the ER selectively into the ER wherein the protein functions, Lys-Asp-Glu-Leu (KDEL) sequence residing at the C-terminus of proteins known to be retained in the ER may be attached to the protein at the C-terminus, similarly to the protein of the invention. It has been known that proteins having KDEL sequence at the C-terminus are bind to receptor proteins residing at Golgi body and the ER, and reverse-transported from Golgi body to the ER (Majoul et al., J. Cell Biol., 133, 777-789, 1996). Further, attachment of certain glycopeptides or carbohydrate chains to the protein or binding of biotin provides selectivity in transition to tissues. Specifically, polypeptides may be modified with asialoglycoprotein (Merwin et al.,Bioconjug Chem.,5,612-620,1994) or galactose (Chen et al.,Hum Gene Ther.,5,429-435,1994) to effect protein accumulation specifically at hepatocytes. Similarly, proteins binding to a protein expressed in certain tissues or cells may be biotinylated to form a complex between the biotinylated protein and avidin, thus enhancing an ability to transit to tissues (Saito et al., Proc. Natl. Acad. Sci. USA, 92, 10227-10231, 1995, Pardridge et al., Pharm. Res., 11, 738-746, 1994).

Although the dose to be administered may be adjusted as appropriate depending on, for example, the property of cancer cells, the age and the body weight of the particular patient, the dose is usually 0.001 mg/kg/administration to 1000 mg/kg/administration. It is preferred to administer such dose every day at the initial stage, and subsequently, every several days to every several months. Dosage form may be modified, and intra-arterial injection, intravenous injection, intramuscular injection, and topical injection to cancer tissues are possible.

Compositions for gene therapy comprising the DNA of the present invention achieve production of the WAR-1 protein in a large amount within the cells, and, against the cancer cells, enable to inhibit the proliferation of cancer cells.

When the composition for gene therapy comprising the DNA of the present invention is introduced into cancer cells, the dosage form may be classified into two types, namely, the case wherein non-viral vectors are used and the case wherein viral vectors are used.

The details of procedures for allowing a DNA of the invention to act within the cells are shown below.

### A. When non-viral vectors are used

Transformations of recombinant expression vectors into cells may be achieved by introducing the DNA of the invention into gene-expression vectors, and transferring the DNA molecules into the cells according to calcium-phosphate coprecipitation, methods for transferring DNA molecules using liposome (the liposome method, Lipofectin method, Lipofectamine method), electroporation, microinjection, methods for transferring DNA molecules into cells along with carriers by particle guns, and the like. In this context, expression vectors used include pBK-CMV, pCAGGS, pcDNA3.1, and pZeoSV.

### B. When viral vectors are used

In this case, methods wherein viral vectors such as adenovirus and retrovirus are used are typical. Specifically, DNA or RNA viruses such as avirulent retrovirus, adenovirus, adeno-associated virus, herpesvirus, vaccinia virus, poxvirus, poliovirus, Sindbis virus, Sendai virus, SV40, human immunodeficiency virus are incorporated with the DNA of the invention, and the recombinant viruses are infected to cells, thus being capable of transforming the gene into cells.

Among the viral vectors, adenovirus vector system is preferably used since adenovirus is known to be extremely higher than other viral vectors in terms of infection efficiency.

Any one of the above methods may be used to introduce the gene encoding WAR-1 into cancer cells. In gene therapy using non-viral vectors, it is preferred to target the gene to the vicinity of an affected area by topical administration, combination with dosage form for enhancing ability to transit tissues, or the like, whereas, in gene therapy using viral vectors, topical administration is not necessary, and intravenous administration may be applicable. Dosage form may be formulations such as liquid formulation, and may be supplemented with conventional carriers if necessary. To facilitate the transfer of the gene into the vicinity of an affected area, sustained-release formulations may be applicable.

In order to allow a gene of the present invention to act as a medicine in practice, one can use an *in vivo* method in which DNA is directly introduced into the body, or an *ex vivo* method in which certain cells are removed from human body, and after transferring the DNA into said cells extracorporeally, the cells are reintroduced into the body (*Nikkei-Science*, April, 1994, pp. 20-45; *Gekkan-Yakuji*, 36(1), 23-48 (1994); *Jikkenn-Igaku-Zokan*, 12(15), 1994). An *in vivo* method is more preferred in the present invention.

Although the amount of a DNA of the present invention in the formulations may vary appropriately depending on the disease to be treated, the age and weight of the patient, and the like, it is typical to administer 0.0001-100 mg, preferably 0.001-10 mg, of a DNA of the present invention every several days to every several months.

In the second aspect, the present invention provides a composition for facilitating neurotrophic factor secretions, which comprises as an active ingredient a WAR-1 protein, or a DNA encoding WAR-1.

The term "composition for facilitating neurotrophic factor secretions" means a pharmaceutical composition having a facilitatory effect on neurotrophic factor secretions from nerve cells that are contacted to said composition. The term "neurotrophic factor(s)" is a common name of proteins having physiological actions such as neuronal survival and maintenance, and neural differentiation, like nerve growth factor (NGF) that was discovered in 1950.

DNAs encoding the proteins of the present invention include, but not limited to, a DNA encoding WAR-1 protein, and a DNA similar to the DNA encoding WAR-1, which encodes a protein having a facilitatory effect on neurotrophic factor secretions. Specific examples are DNAs of the following (1) to (3).
1) a DNA encoding a protein comprising the amino acid sequence of SEQ ID: No. 2 or SEQ ID: No. 4, or a DNA comprising the base sequence of SEQ ID: No. 1 or SEQ ID: No. 3;
2) a DNA encoding a protein comprising an amino acid sequence containing deletion, substitution and/or addition of one or more amino acid residues in the amino acid sequence of SEQ ID: No. 2 or SEQ ID: No. 4, said protein having a facilitatory effect on neurotrophic factor secretions; and
3) a DNA that hybridizes with a DNA comprising the base sequence of SEQ ID: No. 1 or SEQ ID: No. 3 under stringent conditions, and encodes a protein having a facilitatory effect on neurotrophic factor secretions.

The details of these DNAs are as shown above except that biological activity of a protein encoded by the DNA is a facilitatory effect on neurotrophic factor secretions rather than an inhibitory effect on cancer cell proliferations.

Determination whether or not DNAs encode a protein having a facilitatory effect on neurotrophic factor secretions may be conducted by examining a neurotrophic factor expressed in cells introduced with the DNAs, or by examining a neurotrophic factor extracellularly released. For example, the following procedures may be used in the determination.

For biochemical analysis, Western blotting, ELISA and RIA wherein antibodies that recognize diverse neurotrophic factors are used are convenient. Also, electron microscope techniques may be combined with immunohistochemical staining using such antibodies to check the accumulation of neurotrophic factors in the ER. This approach is based on presumption that proteins freshly translated in the cytoplasm stably inhabit in the ER wherein proteinases are in low levels when the proteins are bound to signal recognition proteins or WAR-1 to form a rough ER, but otherwise are subjected to decomposition by proteinases that exist in a large level in the cytoplasm.

Additionally, biologically analytic approach may be used to examine if neurotrophic factors having neurite extension effects are accumulated in the culture supernatant of transformants receiving gene transfer. Specifically, the culture supernatant of transformants is contacted to PC12 cells derived from rat adrenal melanocytoma that are recognized to extend their neurite by the supplement with diverse neurotrophic factors, and then determination is conducted if the accumulation level of neurotrophic factors having neurite extension effects in the transformants is higher than that of control. PC12 cells are available from Dainippon Pharmaceutical Co. Ltd. or Rikenn GeneBank. For the first choice, the biological analysis is more preferred.

In the case that neurite extension effects are recognized, biochemical analysis is required for the next step to examine which neurotrophic factors increase. At this time, many antibodies are necessary to detect diverse neurotrophic factors, but, in this case, there is any possibility to detect unknown factors.

In order to confirm if unknown factors exist, the culture supernatant may be treated with neutralizing antibodies that bind to neurotrophic factors or receptors for them to cause disappearance of their activities, thus observing the lowered effect of neurite extension. When significant neurite extension effects are remained even in the presence of neutralizing antibodies, it may show the presence of unknown neurotrophic factors.

Additionally, cells transferred with a gene for certain neurotrophic factor or cells known to release certain neurotrophic factors may be used in a method for checking that neurotrophic factor secretion is facilitated by enhancing the expression level of the WAR-1 gene. More conveniently, it is preferred that the latter cells, especially cell lines from cancers, are used.

As cells releasing certain neurotrophic factors, T93G cells that are glioblastoma derived from human astrocyte may be used. T93G is available from Dainippon Pharmaceutical Co. Ltd. Diverse factors such as NGF (Emmett et al., Neurochem. Int., 30, 465-474, 1997), TGF-β1, β2 (Naganuma et al., Neurol Med Chir(Tokyo), 36,789-795, 1996), PDGF-B (Potopova etal., Int. J. Cancer, 66,669-677, 1996), bFGF (Takahashi et al., FEBS Lett., 288, 65-71, 1991), IGF-1 (Ambrose et al., J. Cell Physiol., 159,92-100,1994) are known as neurotrophic factors released from T93G cell. Neurotrophic factor secretions facilitated by the increased production of WAR-1 protein have been demonstrated by biological analysis wherein WAR-1 gene is transferred and expressed in T98G cells that is recognized to release diverse neurotrophic factors as shown above, and wherein the neurite extension effect of culture supernatant of the resultant transformants on PC12 cells is used as indicator.

In accordance with the above procedures, proteins may be expressed and prepared starting from the DNA of the invention by culturing continuously the cells transformed with a DNA of the invention under an appropriate condition. In this context, the term "appropriate condition" refers to a condition wherein a cultivation is conducted at 37 °C under 5%CO₂ in a culture medium suitable for respective host cell.

From the transformants thus cultured in an appropriate condition, the proteins of the present invention as described above may be isolated and purified. In this connection, preparation of a crude extract of the present proteins, and purification of the present proteins may be conducted according to, for example, methods as described in "Shinseikagakujikkenn-koza 1, Tannpakushitsu I-bunnri, seisei, seishitsu", Japanese Biochemical Society Ed. 1990.

Specific examples of the proteins of the present invention obtainable as shown above include rat WAR-1 comprising the amino acid sequence of SEQ ID: No. 2, and human WAR-1 comprising the amino acid sequence of SEQ ID: No. 4.

Facilitatory effect of the proteins of the present invention on neurotrophic factor secretions may be determined as described below.

The protein of the present invention is added to the culture of cells such as human glioblastoma line T98G. At this time, the protein is encapsulated into liposome or bound to lipid in order to enhance the permeability across the cell membrane. Alternatively, the endoplasmic reticulum-retained sequence, Lys-Asp-Glu-Leu (KDEL), is attached to the C-terminus of the protein in order to make it possible that the protein migrated to the cytoplasm be transported into the ER. The protein thus treated is added to the culture, and the culture is incubated for several days, so as to give culture supernatant. Facilitatory effect of the resultant culture supernatant on secretion of neurotrophic factor is estimated in accordance with a similar procedure to the above manner wherein the facilitatory effect of DNA is estimated.

Compositions for facilitating neurotrophic factor secretions according to the invention may comprise a substance for enhancing the expression level of WAR-1 gene or a substance for enhancing the production level of WAR-1 protein. Determination of facilitatory effect of such substance on neurotrophic factor secretions may be conducted by the following methods.

Selection for a substance for enhancing the expression level of WAR-1 gene or a substance for enhancing the production level of WAR-1 protein may be conducted directly by examining the accumulation of WAR-1 protein within cells using antibodies directed to WAR-1 protein. The detection may be conducted by attaching a tag sequence to WAR-1 protein, and using an anti-Tag antibody.

Additionally, the detection may be conducted by examining the accumulation of certain secretory protein or cell membrane protein factors related to WAR-1 in the ER. When using secretions of certain secretory protein factors as indicator, ELISA, etc., may be used to examine the accumulation of the proteins in culture supernatant of cells. Further, When using accumulation of certain cell membrane protein factors at cell membrane as indicator, the accumulation may be examined by Western blotting or FACS analysis using antibodies against these factors, or on the basis of increased binding capability to certain ligands if these factors are receptors for the ligands.

Estimation for effects on cells is also applicable. For example, biological effects such as a neurite extension effect of factors that are released from certain cells affected with the above substance and that are accumulated in culture supernatant may be also used in the estimation as described herein.

Alternatively, determination whether or not gene expression level is enhanced may be conducted directly by examining enhanced expression of WAR-1 gene via Northern blotting or RT-PCR. Further, effects of the substances may be also examined by use of activity of reporter gene as indicator by introducing into certain cells expression vectors wherein 5'-upstream sequence containing the promoter region of WAR-1 gene is attached to reporter genes such as lacZ, the luciferase gene, and GFP.

DNAs or proteins of the present invention may be used as active ingredients in pharmaceutical compositions for treating neurodegenerative disorders since they have facilitatory effects on neurotrophic factor secretions. Neurodegenerative disorders are diseases associated with neuro-degeneration or deficiency that are affected by complex actions of an oxidative stress, a neurotoxin, or a genetic factor, and specifically include Alzheimer's disease, cerebral infarction dementia, Parkinson's disease, Huntington disease, and ALS.

Pharmaceutical compositions for treating neurodegenerative disorders according to the present invention may be used as (1) pharmaceutical compositions comprising WAR-1 protein itself or a portion thereof as an active ingredient, (2) may be used as compositions for gene therapy that lead to expression in body of the DNA encoding WAR-1 that is administered to patients as an active ingredient, or (3) may comprise as an active ingredient a substance for enhancing the expression level of WAR-1 gene or the production level of WAR-1 polypeptide.

Target cells of the pharmaceutical compositions for treating neurodegenerative disorders according to the present invention may be either glial cells or nerve cells, although neurotrophic factors responsible for neuronal survival and maintenance is released from glial cells to act on nerve cells in a paracrine fashion, or is released from nerve cells to act in an autocrine fashion.
(1) Pharmaceutical compositions for treating neurodegenerative disorders which comprise WAR-1 protein itself or a portion thereof as an active ingredient:

The pharmaceutical compositions which comprise WAR-1 protein itself or a portion thereof as an active ingredient may be administered in a similar manner to the composition for inhibiting cancer cell proliferation comprising WAR-1 protein as an active ingredient as described above; and
(2) Pharmaceutical compositions for treating neurodegenerative disorders which comprise the DNA encoding WAR-1 as an active ingredient:

When the compositions are used as compositions for gene therapy, WAR-1 polypeptide may be produced in a large amount within glial cells or nerve cells resided in neurodegenerative tissues, and thus releases of neurotrophic factor may be facilitated.

When the composition for gene therapy comprising the DNA of WAR-1 is administered, the dosage form may be classified into two types, namely, the manner wherein non-viral vectors are used and the manner wherein viral vectors are used. The details are similar to those of the case wherein the compositions for inhibiting proliferation of cancer cells are used.

Among the viral vectors, adenovirus vector system is most preferably used in the pharmaceutical compositions for treating neurodegenerative disorders since adenovirus is known to be extremely higher than other viral vectors in terms of infection efficiency, and to express foreign genes in even nondividing cells such as nerve cells.

Sendai virus vector system recognized to infect to nerve cells, and herpesvirus vector system when targeting nerve cells are preferred ones next to adenovirus vector system.

Although the amount of the DNA in the formulations may vary appropriately depending on the disease to be treated, the age and weight of the patient, and the like, it is typical to administer 0.0001-100 mg, preferably 0.001-10 mg, of a DNA of the present invention every several days to every several months.
(3) Pharmaceutical compositions for treating neurodegenerative disorders which comprise as an active ingredient a substance for enhancing the expression level of WAR-1 gene or the production level of WAR-1 polypeptide:

Not only pharmaceutical compositions for treating neurodegenerative disorders which comprise the DNA or the protein of the present invention as an active ingredient, but also the compositions which comprise as an active ingredient a factor or a compound inducing the expression of WAR-1 gene in living bodies or the production of the protein are found to provide facilitatory effects on neurotrophic factor secretions. Accordingly, pharmaceutical compositions for treating neurodegenerative disorders which comprise as an active ingredient such substances as those facilitating the WAR-1 gene expression, or such substances as those facilitating the WAR-1 protein production are fallen within the scope of the present invention. Such substances include peptides, analogues thereof, microorganism cultures, synthesized compounds, and the like.

### BRIEF DESCRIPTION OF DRAWINGS

Figure 1 shows the homology comparisons among the base sequences of cDNAs encoding TRAM from human (HTRAM in the figure), KIAA0057 from human (KIAA0057 in the figure), WAR-1 from human (HWAR1 in the figure), and WAR-1 from rat (RWAR1 in the figure).

Figure 2 shows the homology comparisons among the amino acid sequences deduced from the base sequences of cDNAs encoding TRAM from human (HTRAM in the figure), KIAA0057 from human (KIAA0057 in the figure), WAR-1 from human (HWAR1 in the figure), and WAR-1 from rat (RWAR1 in the figure).

Figure 3 shows the photograph of the electrophoretic profiles analyzed by RT-PCR of the expression of hWAR-1 gene in various cancer cell lines from human. Upper lane shows the RT-PCR result of hWAR-1, whereas the lower lane shows the RT-PCR result of hTRAM.

Figure 4 shows the photograph of the electrophoretic profiles analyzed by Northern hybridization of the expression of hWAR-1 gene in various human tissues. Numbers indicated at the right side show size markers of RNA molecular weight (Kb).

Figure 5 shows the photograph of the electrophoretic profiles analyzed by Northern hybridization of the expression of hTRAM gene in various human tissues. Numbers indicated at the right side show size markers of RNA molecular weight (Kb).

Figure 6 shows the photomicrography of the morphologic changes of human glioblastoma, T98G, infected with the adenovirus incorporated with hWAR-1 gene. A: cells infected with no adenovirus, B: adenovirus-infected cells in which only adenovirus vector is infected, C: cells infected with recombinant adenovirus expressing the hWAR-1 sense strand, and D: cells infected with recombinant adenovirus expressing the hWAR-1 antisense strand.

Figure 7 shows the photomicrography of the morphologic change of T98G cells infected with the adenovirus vector, AxCAwt, with the time course. A: immediately after the infection, B: eight hours after the infection, C: 21 hours after the infection (after about one day), D: 31 hours after the infection (after about 1.5 days), E: 46 hours after the infection (after about two days), and F: 70 hours after the infection (after about three days).

Figure 8 shows the photomicrography of the morphologic changes of T98G cells infected with the recombinant adenovirus vector, AXCAWAR1-L, expressing the sense strand of hWAR-1, with the time course. A: immediately after the infection, B: eight hours after the infection, C: 21 hours after the infection (after about one day), D: 31 hours after the infection (after about 1.5 days), E: 46 hours after the infection (after about two days), and F: 70 hours after the infection (after about three days).

Figure 9 shows the photograph of the electrophoretic profiles analyzed by Northern hybridization using oligonucleotide to detect rWAR-1 gene from rat, which shows the expression of the rWAR-1 gene in various rat tissues. Two bands of the approximately 2.4 Kb transcription products were detected in the tissues wherein the gene was expressed.

Figure 10 shows the photograph of the electrophoretic profiles analyzed by Northern hybridization using, as probe, 1.9Kb EcoRI fragment to detect rWAR-1 gene from rat. Lane 1: juvenile rat hippocampus poly(A) mRNA; Lane 2: adult rat brain poly(A) mRNA; Lane 3: rat retina poly(A) mRNA.

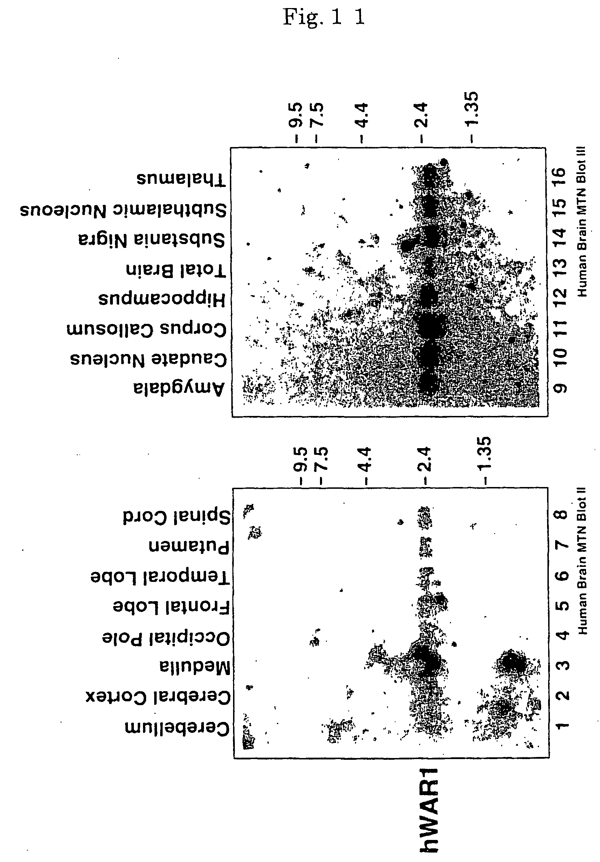

Figure 11 shows the photograph of the electrophoretic profiles analyzed by Northern hybridization of the poly(A) mRNA prepared from various sites of human brain using as probe the DNA fragment detecting specifically hWAR-1 gene from human. Numbers indicated at the right side show size markers of RNA molecular weight (Kb).

### EXAMPLES

The present invention is further illustrated by the following examples, but is not restricted by these examples in any way.

### Example 1

### Preparation of rat cDNA library

In order to clone various new cDNAs, cDNA library was prepared from juvenile rat as shown below.

First of all, tissues had been removed from juvenile rats aged 12 days after birth were homogenized before adding guanidine thiocyanate, and then cesium chloride density gradient centrifugation was performed on the homogenate to prepare 2 mg of total RNA in usual manners.

The total RNA was applied to oligo(dT) cellulose column (Pharmacia) to purify 103 µg of mRNA having a poly(A) supplemental sequence. Single-stranded complementary DNA was synthesized with reverse transcriptase using oligo(dT) primer and random primers, based on 16 µg aliquot of the mRNA. Then, RNaseH and E.coli DNA polymerase I was reacted with the DNA to synthesize double-stranded cDNA. The double-stranded cDNA was blunt-ended by treating it with T4 DNA polymerase, and EcoRI-adaptors were attached to the cDNA at both terminuses. Final amounts of the cDNA when using the oligo(dT) primer and the random primers were 2 µg and 1.3 µg, respectively. A λ phage vector, λgt10, that had been incorporated with the aliquot of the cDNA at the EcoRI cleavage site was introduced into λ phage particles with an *in vitro* packaging kit (Stratagene), and infected to *E*. *coli* C600^{hfl} (Stratagene) to prepare the intended cDNA library of juvenile rat. Plaque forming units (pfu) of the cDNA synthesized with oligo(dT) primer and random primers were 8.8X10⁷ pfu and 2.5X10⁷ pfu per µg, respectively.

### Example 2

### Determination of the base sequence of cDNA encoding rWAR-1

λ phage DNAs were recovered from clones randomly selected from the cDNA library prepared in Example 1 by the usual plate lysine method, and cleaved with restriction enzyme EcoRI, followed by subcloning the inserted cDNA portions into M13 phage vector. Base sequence of the inserted cDNA portion in one of them was determined, revealing that the cloned cDNA is approximately 2.2 Kb in length, and has an open reading frame spanning approximately 1 Kb. This was predicted to be a full length cDNA in consideration of the poly(A) sequence length since the mRNA corresponding to the cDNA was estimated approximately 2.4 Kb in size according to Northern analysis using the 1.8 Kb EcoRI fragment as probe in usual manners. The resultant clone was named clone 12. Search of the base sequence of cDNA of clone 12 on GenBank databases revealed that the cDNA of clone 12 is novel since no sequence that has the identical sequences was searched, although it shares 59.7% homology in terms of base sequence and 57.0% homology in terms of amino acid sequence with TRAM that is an ER membrane transport-associated protein from human (GenBank Accession No. X63679), whereas it shares 53.7% homology in terms of base sequence and 41.0% homology in terms of amino acid sequence with KIAA0057 (GenBank Accession No. D31762) (Table 1). The protein encoded by the new cDNA was named rat WAR-1 (rWAR-1). Base sequence and deduced amino acid sequence of rWRA-1 are shown in SEQ ID: Nos. 1 and 2. Additionally, the base sequence and the amino acid sequence of known human TRAM (hTRAM) are shown in SEQ ID: Nos. 5 and 6, and the base sequence and the amino acid sequence of human KIAA0057 are shown in SEQ ID: Nos. 12 and 13.

**Table 1**

| % Homology between human WAR-1, human TRAM, rat WAR-1 and KIAA0057 | | | | | |
|---|---|---|---|---|---|
| | | Human WAR- 1 | Rat WAR- 1 | Human TRAM | KIAA 0057 |
| Human | base | | 72.4 | 76.3 | 64.1 |
| WAR-1 | sequence | | (72.7) | (70.7) | (44.1) |
| | (amino acid | | | | |
| | sequence) | | | | |
| Rat | base | 72.4 | | 59.7 | 53.7 |
| WAR-1 | sequence | (72.7) | | (57.0) | (41.0) |
| | (amino acid | | | | |
| | sequence) | | | | |
| Human | base | 76.3 | 59.7 | | 69.3 |
| TRAM | sequence | (70.7) | (57.0) | | (51.3) |
| | (amino acid | | | | |
| | sequence) | | | | |
| KIAA | base | 64.1 | 53.7 | 69.3 | |
| 0057 | sequence | (44.1) | (41.0) | (51.3) | |
| | (amino acid | | | | |
| | sequence) | | | | |

*E. coli* DH5ₐ (prWAR-1) that contains prWAR-1 wherein the cDNA fragment of rWAR-1 as described above is incorporated into a vector pBluescript II was deposited at The National Institute of Bioscience and Human Technology, Agency of Industrial Science and Technology, 1-1-3 Higashi, Tsukuba, Ibaraki, Japan (description of microorganism: *E*. *coli* DH5ₐ (prWAR-1); deposition date: October 6, 1998; deposit number: FERM P-17018).

### Example 3

### Cloning of cDNA encoding hWAR-1 and determination of its base sequence

Clone having the cDNA of human type WAR-1 was screened from human cDNA library (Clontech) in usual manners using as probe the 0.8 Kb EcoRI-XhoI DNA fragment corresponding to the open reading frame of rWAR-1. As a result, one clone was isolated from the human cDNA library at 1x10⁶ pfu. Determination of the base sequence revealed that the base sequence shares 72.4% homology and the amino acid sequence shares 72.7% homology with those of raWAR-1 as obtained in Example 2, concluding that this should be cDNA encoding human type WAR-1 (hWAR-1) (Table 1). The determined base sequence and the deduced amino acid sequence are shown in SEQ ID: Nos. 3 and 4. The hWAR-1 shares 76.3% homology in terms of base sequence and 70.7% homology in terms of amino acid sequence with TRAM as mentioned above, whereas it shares 64.1% homology in terms of base sequence and 44.1% homology in terms of amino acid sequence with KIAA0057 (Table 1).

*E*. *coli* DH5ₐ (phWAR-1) that contains phWAR-1 wherein the cDNA fragment of hWAR-1 as described above is incorporated into a vector pBluescript II was deposited at The National Institute of Bioscience and Human Technology, Agency of Industrial Science and Technology, 1-1-3 Higashi, Tsukuba, Ibaraki, Japan (description of microorganism: *E*. *coli* DH5ₐ (phWAR-1); deposition date: October 6, 1998; deposit number: FERM P-17019).

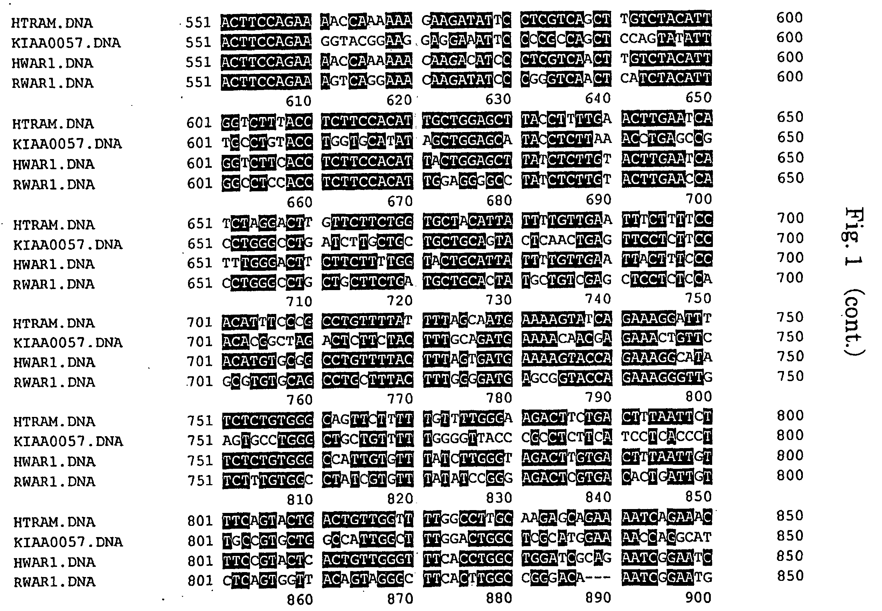

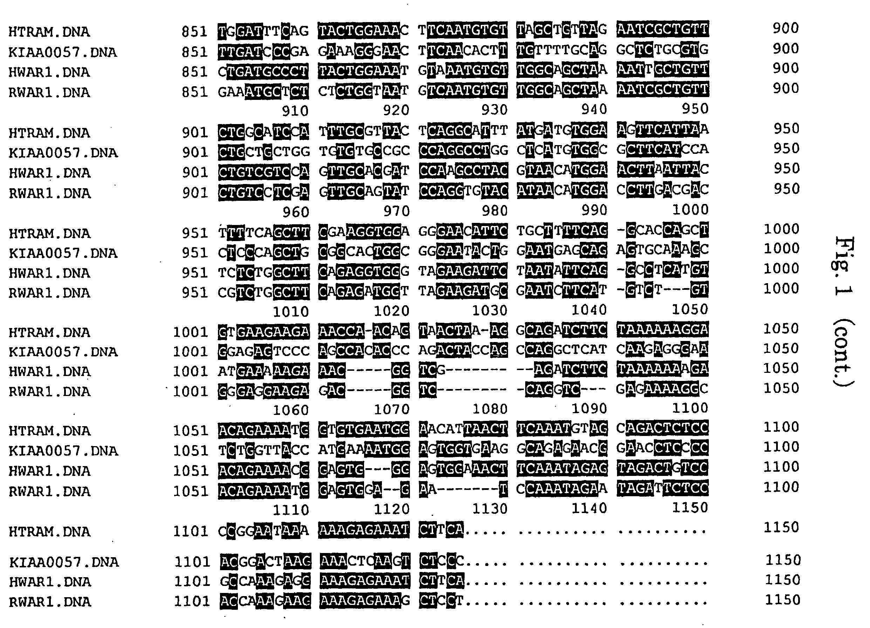

Comparison between the base sequences of cDNAs encoding hWAR-1, rWAR-1, hTRAM and KIAA0057 is shown in Figure 1. Comparison between the amino acid sequences of hWAR-1, rWAR-1, hTRAM and KIAA0057 is shown in Figure 2.

### Example 4

### Examination of expression of the gene encoding hWAR-1 in various human cancer established cell lines

For the purpose of assessment of expression of the genes encoding hWAR-1 and hTRAM in different types of human cancer cell, primers for each of specific amplifications of hWAR-1 and hTRAM mRNAs were designed to subject to RT-PCR under the following conditions.

For hWAR-1 mRNA amplification, the segments corresponding to positions 823-846 (SEQ ID NO:7) and the positions 1093-1116 (SEQ ID NO:8) of the hWAR-1 base sequence depicted in Figure 1 were used as 5' and 3' primers, respectively. For hTRAM mRNA amplification, the segments corresponding to positions 823-846 (SEQ ID NO:9) and positions 1087-1110 (SEQ ID NO:10) of the hTRAM base sequence depicted in Figure 1 were used as 5' and 3' primers, respectively.

As human cancer cell lines, uterocervical cancer cell line Hela, lung cancer cell line A549, bladder carcinoma cell line T24, large bowel cancer cell line SW480, glioblastoma cell line T98G, hepatoma cell line HepG2, Wilms' tumor: renal carcinoma cell line G401, Burkitt lymphoma: B lymphoma cell line Daudi, and T lymphoma cell line MOLT4, Jurkat were used. These cell lines are all available from DAINIPPON PHARMACEUTICAL CO., LTD., except for T24 cell line that is available from RIKEN GENE BANK.

RT-PCR reaction was conducted as follows.

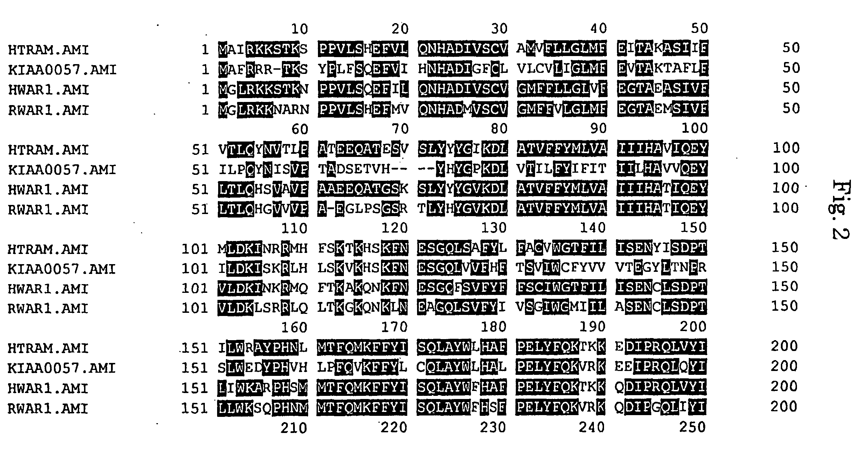

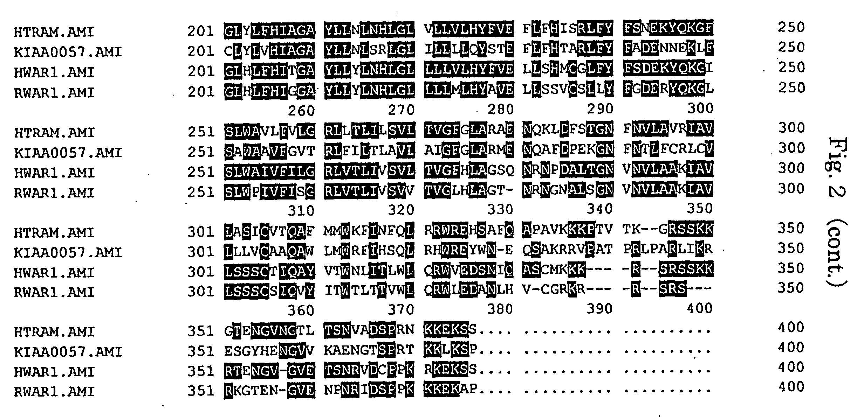

Firstly, total RNA was prepared from each cell line described above using a total RNA purification kit (NIPPON GENE CO., LTD.). To 4 µg (9.5 µl) of the total RNA, 2.0 µl of 100mM DTT, 4.0 µl of 5 x First Standard Buffer (Gibco BRL), 0.5 µl of 40 U/µl RNasein (Promega), 2.0 µl of 10mM dNTP, 1.0 µl of 0.2 µg/µl pd(N)₆ primer, 1.0 µl of 200 U/µl MMTV-RT (Gibco BRL) were added, and the mixture was warmed to 37°C for 45 minutes, followed by heated to 95°C for 5 minutes to inactivate the enzymes. Then, a 16 µl aliquot was removed from the reaction mixture and added with 24 µl of H₂O. To 2.0 µl of this mixture, 2.5 µl of 10XPCR Buffer (Perkin Elmer), 2.5 µl of 2.0mM dNTP, 25 µM of each 5' primer, 25 µM of each 3'primer, 16.875 µl of H₂O and 0.125 µl of 5 U/µl AmpliTaq Gold (Perkin Elmer) were added. The mixture was heated to 95°C for 9 minutes, and was subjected to 35 cycles of the reactions at 95°C for 1 minute, 60°C for 1 minute and 72°C for 2 minutes. Finally, the mixture was heated to 72°C for 10 minutes, and then 3 µl of this mixture was subjected to 2% agarose gel electrophoresis. The results are shown in Figure 3.

Expression of hTRAM was observed in all of these cancer cell lines, and that expression of hWAR-1 was shown to be somewhat lower in Jurkat etc. than that of hTRAM, indicating that hWAR-1 gene differs from hTRAM gene (Figure 3) in terms of expression manner.

### Example 5

### Examination of expression in normal human tissue of the gene encoding hWAR-1

In order to examine expressions of hWAR-1 and hTRAM genes in tissues, the following experiments were conducted.

First, the hWAR-1 gene fragment of interest was amplified by means of RT-PCR as described in Example 4 using the above primer sequence specific for hWAR-1 (SEQ ID NOs:7 and 8), and the amplified fragments were cloned to pT7Blue(R)T vector (Novagen). Like this, the hTRAM gene fragment of interest was amplified by means of RT-PCR using the primer sequence specific for hTRAM (SEQ ID NOs:9 and 10) and cloned to the vector. RT-PCR was conducted using these plasmids as template and the primer sequences (SEQ ID NOs:9 and 10), and the amplified DNA fragments were then collected by polyacrylamide gel electrophoresis.

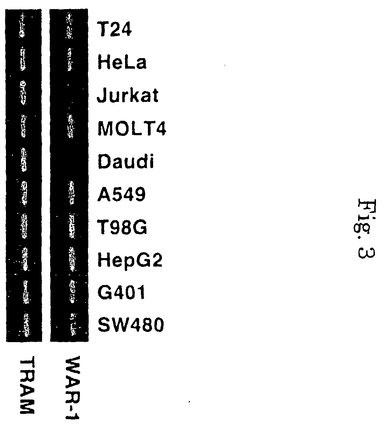

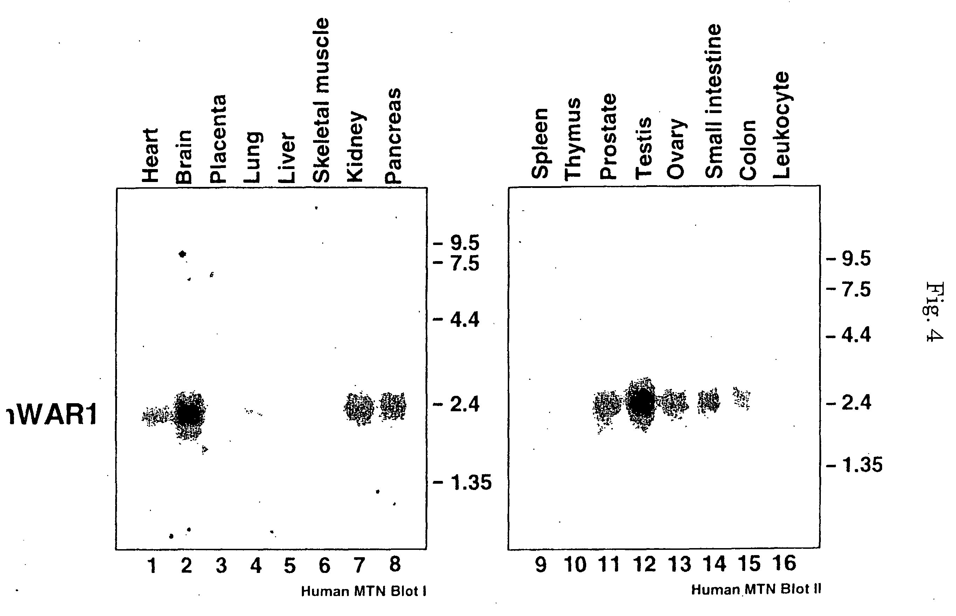

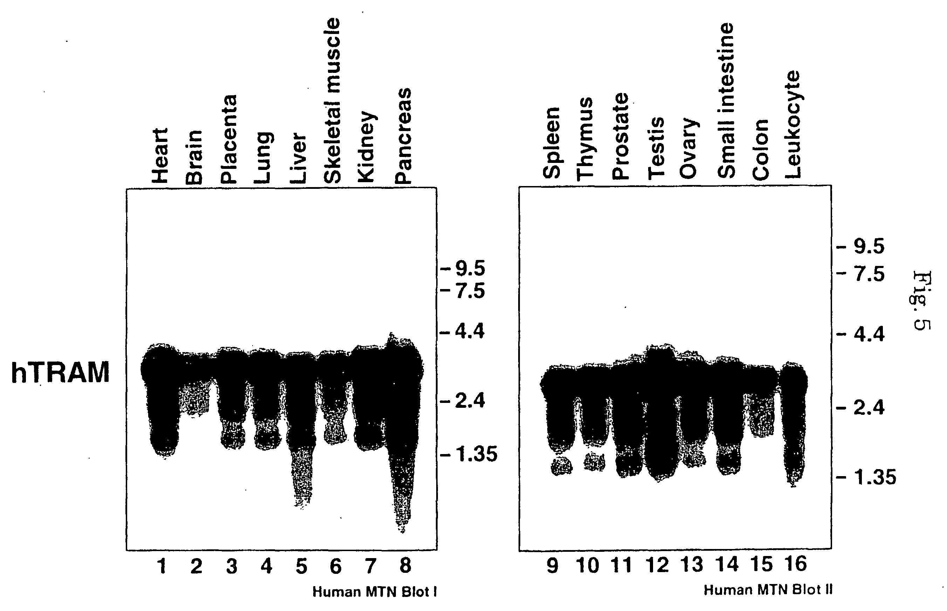

Subsequently, resultant DNA fragments were labeled with ³²P according to multiprime labeling method to prepare probes, and were hybridized onto MTN blot filter purchased from CLONTECH Laboratories, Ltd. (human MTN blot I and human MTN blot II) according to conventional techniques. β-actin (CLONTECH Laboratories, Ltd.) was used as a control probe. The results of hWAR-1 and TRAM gene expressions in tissues are shown in Figures 4 and 5, respectively. Expression of hTRAM was observed in all of the tissues (Figure 5), whereas expression of hWAR-1 was not observed in tissues such as lymphatic system (spleen, thymus and lymphocyte), lung and liver (Figure 4). Based on the results of Example 4 showing that expression of hTRAM gene was observed in the cell lines from lung cancer (A459), T lymphoma (MOLT4, Jurkat) and hepatoma (HepG2) (Figure 3), expression of hTRAM in these tissues was shown to develop in association with malignant transformation.

Intensity of the bands obtained in the autoradiogram was analyzed to examine the expression level in each organ, comparing with the intensity of β-actin as standard. The results of this examination are shown in Table 2. Values shown in the table has been standardized for the expression in testis as 1.00.

**Table 2**

| Tissue | hWAR- 1/ β-actin | Tissue | hWAR-1/ β-actin |
|---|---|---|---|
| Heart | 0.56 | Spleen | 0.00 |
| Brain | 1.47 | Thymus | 0.00 |
| Placenta | 0.00 | Prostate | 0.28 |
| Lung | 0.00 | Testis | 1.00 |
| Liver | 0.00 | Ovary | 0.25 |
| Skeletal muscle | 0.00 | Small intestine | 0.15 |
| Kidney | 0.74 | Colon | 0.12 |
| Pancreas | 1.21 | Peripheral | 0.00 |
| | | leukocyte | |

Table 2 apparently shows that hWAR-1 gene was expressed most highly in brain, similar to rWAR-1 gene, and was expressed more highly in pancreas, kidney and testis.

### Example 6

### Construction of recombinant cosmid vector

Cosmid vectors incorporated with the sense or antisense strand of hWAR-1 were constructed using the approximately 1.3 Kb DNA fragment of the cDNA encoding hWAR-1 spanning from the *Pvu*II cleavage site located 36 base pairs upstream of the ATG initiation codon to *Dra*I cleavage site located 139 base pairs downstream of the TAA termination codon. Specifically, said 1.3 Kb *Pvu*II/*Dra*I fragment of hWAR-1 was ligated to a *Swa*I-cleaved pAxCAwt cosmid vector (described in Kanegae et al., 1995, Nucleic Acid Res., 23, 3816-3821 and available as adenovirus expression vector kit form TAKARA SHUZO CO., LTD.). Then, any cosmid vector not containing the insertion sequence was digested with *Swa*I, and an aliquot of the reaction mixture was *in vitro* packaged according to the conventional techniques. From the developed colonies obtained after infection of *E*. *coli* DH5α, cosmid DNAs were recovered, cleaved with *Eco*RI/*Xho*I and the DNA was analyzed on 1% agarose gel electrophoresis. As a result, nine 9 clones expressing the sense strand RNA and six clones expressing the antisense strand RNA were obtained, and, among them, each three clones were selected. Those expressing the sense strand RNA were cleaved with *Eco*RI/*Xba*I or *Bgl*II, and those expressing the antisense strand RNA were cleaved with *Stu*I/*Xba*I or *Eco*RI/*Xho*I to check the direction and the integrity of the inserted DNA fragment.

### Example 7

### Preparation of recombinant adenovirus

A recombinant adenovirus vector wherein the expression unit of hWAR-1 gene (sense/antisense) as constructed in Example 6 was inserted into E1 gene deletion site of a non-proliferative recombinant adenovirus vector derived from human adenovirus type 5 (E1 and E3 genes have been deleted), was prepared according to the following procedures. As promoter, the CAG promoter was used, which is disclosed as a high expression vector in Japanese Patent Publication (kokai) No. 3-168087, and the preparation of the recombinant adenovirus was conducted according to the ordinary techniques (Miyake et al., Proc. Nat1. Acad. Sci., Vo1.93, 1320-1324(1996) and Japanese Patent Publication No. 7-298877).

A virus DNA-terminal protein complex was prepared according to the ordinary technique (Japanese Patent Publication No. 7-298877) from the recombinant adenovirus vector AxCAwt (described in Kanegae et al., 1995, Nucleic Acid Res., 23, 3816-3821 and available as adenovirus expression vector kit form TAKARA SHUZO CO., LTD.) wherein only CAG promoter was inserted into the E1 gene deletion site of the adenovirus, and the complex was digested simultaneously with the restriction enzymes EcoT221 and ClaI. The restriction enzyme-digested virus DNA-terminal protein complex and the cosmid vector inserted with the hWAR-1 sense or antisense strand as prepared in Example 6 were used to transform 293 cells by means of calcium phosphate co-precipitation. After cloning the resultant recombinant adenovirus, the virus DNAs were digested with *Xho*I and *Cla*I to screen the virus of interest, thus obtaining adenovirus vectors AxCAWAR1-L (sense strand) and AxCAWAR1-R (antisense strand). These virus were passaged four times to obtain the virus solution, and the titer of the virus solution was then determined according to a ordinary technique as described in Japanese Patent Publication (kokai) No. 7-298877. In the subsequent experiments, the virus solution was used.

### Example 8

### Expression of hWAR-1 gene by infection of recombinant adenovirus vector

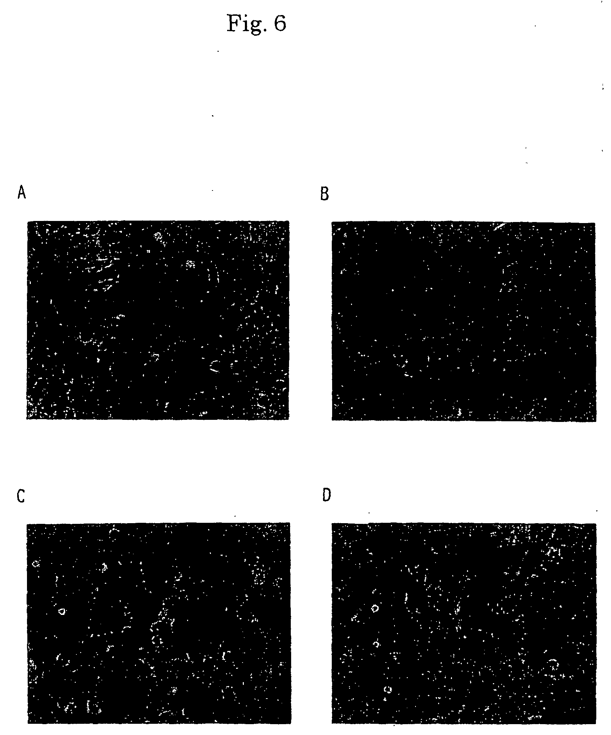

Human glioblastoma cell line T98G was infected at 10 multiplicity of infection with the adenovirus vectors AxCAWAR1-L or AxCAWAR1-R, and AxCAwt (control virus) as prepared in Example 7 at 37°C for 1 hour, and then cultured in a minimum nutrient culture medium containing 5% FCS. On the next day of the infection, the culture medium was replaced with a reduced serum medium (0.5% FCS). On the second day after the infection, all of the cells infected with adenoviruses degenerated in comparison with the cells subjected to infection procedure in culture medium alone, showing that the adenovirus infection affected somewhat. However, remarkable change in cell morphology was observed in the cells infected with AxCAWAR1-L expressing the sense strand (Figure 6). No difference in cell morphology was observed between the cells infected with AxCAwt and the cells infected with AxCAWAR1-R. T98G cells infected with AxCAWAR1-L underwent cell death three days after the infection.

### Example 9

### Morphologic change of T98G cell by WAR-1 sense RNA expression

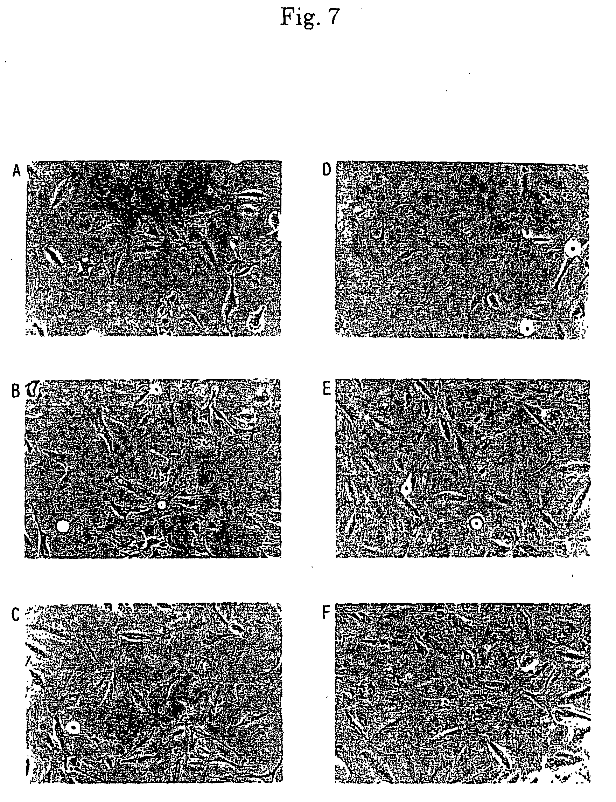

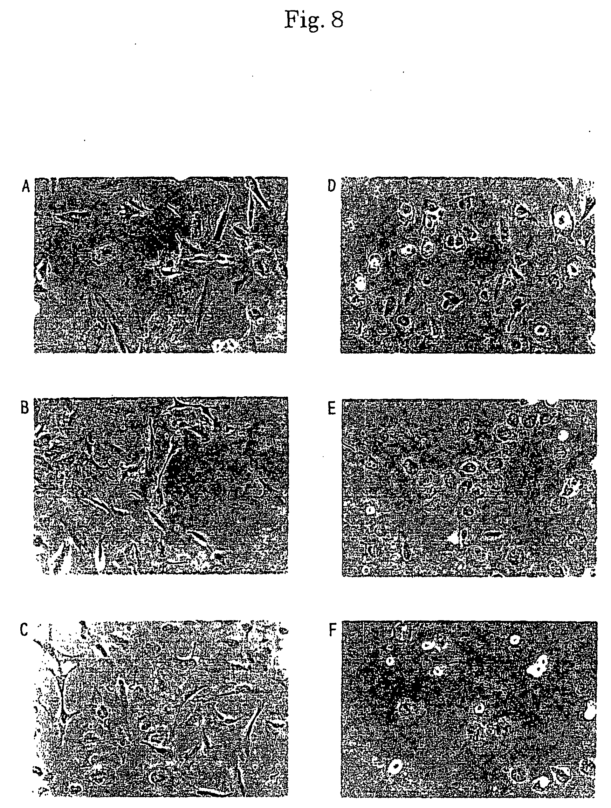

T98G cells were adjusted to a low density (about 1/10 of the confluent density in Example 8) so that morphologic change of the cells could be easily observed, then were infected as described in Example 8 with the adenovirus vector AxCAWAR1-L (sense strand) or AxCAWAR1-R (antisense strand), and AxCAwt (control), and were observed for their morphologic change with time course. On the next day of the infection, even the control cells infected with AxCAwt underwent degeneration relatively to the cells subjected to infection procedure in culture medium alone, showing that the adenovirus infection affected somewhat was found (Figure 7). However, in comparison with the cells infected with AxCAwt, the cells infected with AxCAWAR1-L expressing the hWAR-1 sense strand showed remarkable morphologic change on the next day of the infection (21-31 hours later of the infection), and underwent cell death on the third day after the infection (Figure 8). No morphologic difference between the cells infected with AxCAWAR1-R expressing antisense RNA and the cells infected with AxCAwt was found.

### Example 10

### Morphologic change of T98G cell by addition of WAR-1 protein

WAR-1 protein is previously enclosed into liposome or bound to lipid to enhance its permeability across cell membrane. Endoplasmic reticulum-retained sequence, Lys-Asp-Glu-Leu (KDEL), is attached to the C-terminus of the protein so that the protein incorporated into cytoplasm can be transported to endoplasmic reticulum. The protein of the invention thus treated is added to a culture solution of T98G as used in Examples 8 and 9, and the culture solution is cultured for several days. Thereafter, the cell counts of T98G cells and the morphologic change of the cell can be observed to determine inhibitory effect on cancer cell proliferation. Also, inhibitory effect on cancer cell proliferation can be determined by measuring the decrease in its ability to incorporate ³H-labeled thymidine (Nagase et al., Int. J. Cancer, 65, 620-626, 1996).

### Example 11

### Examination of the expression of rat WAR-1 gene in tissues

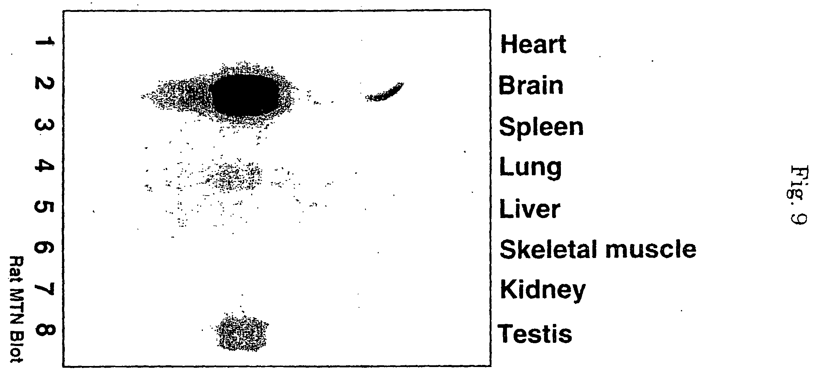

Hybridization using an oligonucleotide probe which specifically detect rWAR- 1 gene was performed onto rat MTN blot filter purchased from CLONTECH. The nucleotide sequence of the probe used in the hybridization was ATTTCTGTGCCTTTCTCGACCTGGACCGTCTCTTCCTCCCACAGACA ATTTTCTGTGCCTTTTCTCGACCTGGACCGTCTCTTCCTCCCACAGACA as described in SEQ ID NO:11. Hybridization conditions were in accordance with the protocol of CLONTECH. As a result of the hybridization, rWAR-1 gene was expressed intensively in brain, which are as shown in Figure 9. Also, the expression of this gene was observed in testis, whereas the expressions in lung and kidney were weak.

### Example 12

### Examination of human and rat WAR-1 expressions in nerve related sites

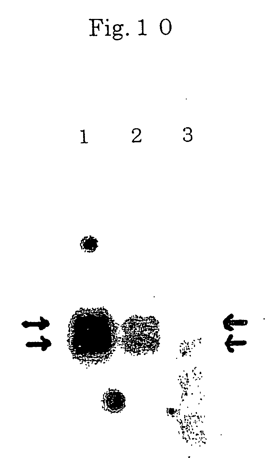

Northern hybridizations were conducted using rWAR-1 gene as a probe with a poly(A)mRNA prepared from jurenile rat hippocampus according to a ordinary method, and with mature rat brain and retina poly(A)mRNAs purchased from CLONTECH. The result shows that expression of rWAR-1 gene was also observed in retina as shown in Figure 10. Decreased intensity in the detected bands were bilieved to be caused by degradation of poly(A)mRNA.

Further, using a probe for detecting hWAR-1 gene as used in Example 5, hybridization was conducted onto MTN blot filter purchased from CLONTECH (human brain MTN blot II and III) (Figure 1). The result shows that expression of hWAR-1 gene was observed in all sites in brain. Expression of the gene was also observed in spinal cord. It suggested that hWAR-1 gene also expressed in peripheral nervous system.

### Example 13

### Assessment of neurite extension effect on PC12 cell using the culture supernatants of the cells infected with adenovirus vectors

Human glioblastoma cell line T98G was infected at 10 multiplicity of infection with the adenovirus vectors AxCAWAR1-L or AxCAWAR1-R, or AxCAwt (control virus) at 37°C for 1 hour and cultured in a minimum nutrient culture medium containing 5% FCS. Same virus infection study was conducted for human lung cancer A549 cell, which is a non-nervous system culture, as a negative control. Supernatant from each culture after incubation for two days was collected and centrifuged, and the culture supernatant was then passed through a 0.22-micron membrane filter to remove cell debris.

Optionally, obtained culture supernatant was fivefold concentrated using Centriprep (3000Kd cut)(Amicon Corporation).

PC12 cells in 100 µl of DMEM-10%FCS medium was plated on a collagen-coated 96-well plate at 1-5 x 10⁴ cells/ml per well, and the plate was incubated for four days. The culture supernatant was discarded, and the supernatants obtained from the cell culture infected with recombinant adenovirus were added up to 100 µl of final volume as shown in Tables 3 and 4. Final serum concentration of the culture was adjusted to 10% FCS. As a sample to be added, culture medium, the culture supernatant of the non-infected cell (Mock), the culture supernatant of the cell infected with AxCAWAR1-L (WAR(+)), and the culture supernatant of the cell infected with AxCAwt (control) were used. 10 µl of 500ng/ml β-NGF was added as a positive control in this assay system.

After addition of a culture supernatant, incubation was continued for two days, and neurite extension effect on PC12 cells was observed microscopically. Potent neurite extension effect was observed only in PC12 cells added with the culture supernatant of T98G cells infected with the adenovirus expressing hWAR-1 sense strand. Neurite extension effect was not found in all experiments using the culture supernatant of A549 cells. The results of Table 4 confirmed that at least 50 µl of the culture supernatant of the cell infected with AxCAWAR1-L was enough to raise neuritic outgrowth effect.

The above results distinctly show that the secretion to extracellular space of the neurotrophic factor produced in T98G cells was enhanced by the expression of WAR-1 gene. The Table 3 showes the results of PC12 assay using the culture supernatants of T98G and A549 cells infected with adenovirus (100 µl of the supernatant from each infected cell was added). In Table 3, "+++", "++", "+" and "-" shows the neurite extension of PC12 cells in order of its magnitude.

**Table 3**

| | β-NGF | T98G | A549 |
|---|---|---|---|
| Medium | - | - | - |
| Mock | | + | - |
| Control | | + | + |
| WAR(-) | | + | + |
| WAR(+) | | ++ | + |
| β-NGF | +++ | | |

Table 4 shows the results of PC12 assay using the culture supernatants of T98G cells infected with adenovirus. In Table 4, "+++", "++", "+", "-/+" and "-" shows the neurite extension of PC12 cells in order of its magnitude.

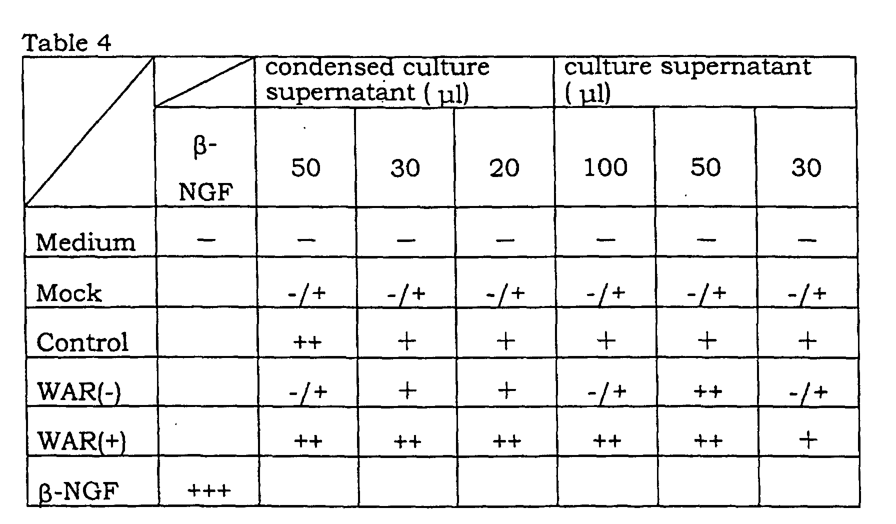

### INDUSTRIAL APPLICABILITY

DNAs of the present invention encode WAR-1 that has a inhibitory effect on cancer cell proliferations, and production or induction therefor of WAR-1 polypeptide encoded by the DNA within cells makes possible inhibition of cancer cell proliferation, which leads to cell death of cancer cells. The invention provides a new therapy for cancers based on such actions of WAR-1.

Additionally, the production or induction therefor makes possible prevention from neuro-deficiency at neurodegenerative areas in central and peripheral nervous systems, and at the same time facilitation of secretions of diverse nerve growth factors useful for activation of cells just before entering into cell death and recovering to normal neurotransmission. The invention provides a new therapy for neurodegenerative diseases.

## Claims

1. A DNA encoding a protein selected from a group consisting of:
(a) a protein comprising the amino acid sequence of SEQ ID: No. 2 or SEQ ID: No. 4, and
(b) a protein comprising an amino acid sequence containing deletion, substitution and/or addition of one or more amino acid residues in the amino acid sequence of SEQ ID: No. 2 or SEQ ID: No. 4, said protein having an inhibitory effect on cancer cell proliferations.

2. A DNA selected from a group consisting of
(c) a DNA comprising the base sequence of SEQ ID: No. 1 or SEQ ID: No. 3, and
(d) a DNA that hybridizes with the DNA of the above (c) under stringent conditions, and encodes a protein having an inhibitory effect on cancer cell proliferations.

3. The DNA of claim 2, which is cloned from chromosomal DNA libraries using all or part of the DNA of SEQ ID: No. 1 or SEQ ID: No. 3 as probe.

4. The DNA of claim 3, which contains a promoter region.

5. The DNA of claim 1 or 2, which is contained in the microorganism of deposit number FERM BP-6910 or FERM BP-6911.

6. A protein obtainable by the expression of the DNA of any one of claims 1 to 5.

7. A recombinant expression vector comprising the DNA of any one of claims 1 to 5.

8. A recombinant adenovirus vector comprising the DNA of any one of claims 1 to 5.

9. A transformant wherein the cell is transformed with the recombinant expression vector of claim 7 or 8.

10. A DNA which is useful as hybridization probe or PCR primer, which is a single- or double-stranded DNA comprising all or pat of the DNA of any one of claims 1 to 5, and which makes possible the specific detection of the expression of a DNA comprising the base sequence of SEQ ID: No. 1 or SEQ ID: No. 3.

11. The DNA of claim 10, which consists of the following sequences:

5' primer sequence; 5'-CACCTGGCTGGATCGCAGAATCGG-3' (SEQ ID:
No. 7)

3' primer sequence; 5'-CTCTTTCCTCTTTGGCGGACAGTC-3' (SEQ ID:
No. 8).

12. A method for detecting the expression of a DNA comprising the base sequence of SEQ ID: No. 1 or SEQ ID: No. 3, which comprises using the DNA of claim 10 or 11 as hybridization probe or PCR primer.

13. An antibody that binds to the protein of claim 6.

14. A method for detecting the expression of a protein comprising the amino acid sequence of SEQ ID: No. 2 or SEQ ID: No. 4, which comprises using the antibody of claim 13.

15. A method for diagnosing cancers, which comprising the method for the detection of claim 12 or 14.

16. A pharmaceutical composition comprising the DNA of any one of claims 1 to 5, or the protein of claim 6 as an active ingredient.

17. A composition for inhibiting proliferation of cancer cells, which is characterized in that the composition enhances the expression level of a DNA comprising the base sequence of SEQ ID: No. 1 or SEQ ID: No. 3.

18. A composition for inhibiting proliferation of cancer cells, which comprises the DNA of any one of claims 1 to 5 as an active ingredient.

19. A composition for inhibiting proliferation of cancer cells of claim 18, which comprises an adenovirus vector.

20. A composition for facilitating neurotrophic factor secretions, which comprises as an active ingredient a DNA encoding a protein selected from a group consisting of:
(a) a protein comprising the amino acid sequence of SEQ ID: No. 2 or SEQ ID: No. 4, and
(b) a protein comprising an amino acid sequence containing deletion, substitution and/or addition of one or more amino acid residues in the amino acid sequence of SEQ ID: No. 2 or SEQ ID: No. 4, said protein having a facilitatory effect on neurotrophic factor secretions.

21. A composition for facilitating neurotrophic factor secretions, which comprises as an active ingredient a DNA selected from a group consisting of:
(c) a DNA comprising the base sequence of SEQ ID: No. 1 or SEQ ID: No. 3, and
(d) a DNA that hybridizes with the DNA of the above (c) under stringent conditions, and encodes a protein having a facilitatory effect on neurotrophic factor secretions.

22. The composition for facilitating neurotrophic factor secretions of claim 21, which comprises as an active ingredient a DNA that is cloned from chromosomal DNA libraries using all or part of the DNA of SEQ ID: No. 1 or SEQ ID: No. 3 as probe.

23. The composition for facilitating neurotrophic factor secretions of claim 22, which comprises as an active ingredient the DNA that contains a promoter region.

24. The composition for facilitating neurotrophic factor secretions of claim 20 or 21, which comprises as an active ingredient a DNA that is contained in the microorganism of deposit number FERM BP-6910, or FERM BP-6911.

25. The composition for facilitating neurotrophic factor secretions of any one of claims 20 to 24, wherein the DNA is comprised in a recombinant expression vector.

26. The composition for facilitating neurotrophic factor secretions of claim 25, wherein the DNA is comprised in an adenovirus vector.

27. A composition for facilitating neurotrophic factor secretions, which comprises as an active ingredient a protein selected from a group consisting of:
(a) a protein comprising the amino acid sequence of SEQ ID: No. 2 or SEQ ID: No. 4, and
(b) a protein comprising an amino acid sequence containing deletion, substitution and/or addition of one or more amino acid residues in the amino acid sequence of SEQ ID: No. 2 or SEQ ID: No. 4, said protein having a facilitatory effect on neurotrophic factor secretions.

28. A composition for facilitating neurotrophic factor secretions, which comprises as an active ingredient a protein encoded by a DNA selected from a group consisting of:
(c) a DNA comprising the base sequence of SEQ ID: No. 1 or SEQ ID: No. 3, and
(d) a DNA that hybridizes with the DNA of the above(c) under stringent conditions, and encodes a protein having a facilitatory effect on neurotrophic factor secretions.

29. The composition for facilitating neurotrophic factor secretions of claim 27 or 28, which comprises as an active ingredient a protein encoded by a DNA that is contained in the microorganism of deposit number FERM BP-6910, or FERM BP-6911.

30. A composition for facilitating neurotrophic factor secretions, which comprises as an active ingredient a substance for enhancing the expression level of the DNA of SEQ ID: No. 1 or SEQ ID: No. 3 or a substance for enhancing the production level of a protein comprising the amino acid sequence of SEQ ID: No. 2 or SEQ ID: No. 4.

31. A pharmaceutical composition for treating neurodegenerative diseases, which comprises the composition for facilitating neurotrophic factor secretions of any one of claims 20 to 30.

32. A method for facilitating secretion of neurotrophic factors, which comprises enhancing the expression level of a DNA comprising the base sequence of SEQ ID: No. 1 or SEQ ID: No. 3, or enhancing the production level of a protein comprising the amino acid sequence of SEQ ID: No. 2 or SEQ ID: No. 4.
